# EUROPEAN PATENT SPECIFICATION

(11) **EP 0 616 701 B1**
(45) Date of publication and mention of the grant of the patent: **13.02.2002**
(21) Application number: 93901377.7
(22) Date of filing: 10.12.1992
(51) Int. Cl.: G03F 7/00, G03F 7/027, C07C 2/04

(54) **PHOTOCURABLE CYCLOBUTARENE COMPOSITIONS**
STRAHLUNGSHÄRTBARE CYCLOBUTAREN ZUSAMMENSETZUNGEN
COMPOSITIONS PHOTODURCISSANTES DE BUTARENE CYCLIQUE

(30) Priority: 10.12.1991 US 805395
(43) Date of publication of application: 28.09.1994
(73) Proprietor: THE DOW CHEMICAL COMPANY, Midland, Michigan 48674 (US)
(72) Inventor: OAKS, Frank L., San Carlos, CA 94070 (US); MOYER, Eric S., Midland, MI 48642 (US); RUTTER, Edward W., Midland, MI 48642 (US); HARRIS, Robert F., Midland, MI 48642 (US)
(74) Representative: Sternagel, Hans-Günther, Dr.
(86) International application number: PCT/US92/10649
(87) International publication number: WO 93/12055

(56) References cited:
- EP-A- 0 527 572
- US-A- 4 106 943
- US-A- 4 540 763
- US-A- 4 565 767
- US-A- 4 724 260
- US-A- 4 743 399
- US-A- 4 759 874
- US-A- 4 864 010
- US-A- 5 041 600
- US-A- 5 156 656
- Journal of Electronic Materials, Vol. 19, No. 12, 1990, "Benzocyclobutene Dielectrics for the Fabrication of High Density, Thin Film Multichip Modules", (BURDEAUX et al.), 1357-1366, especially pages 1357-1358.

## Description

This invention relates to a photocurable organic-soluble mixture comprising at least one oligomerized cyclobutarene.

Such polymers are used in passivation films, photoresists, as insulating layers in fabricating electrical devices and as protective films for semiconductor elements.

Thermal- and/or photo-cured polyimides, which are currently used in many of these applications, have drawbacks, some of which include: a short shelf life, less than desirable thermal oxidative stability, microcracking during thermal cycling, and lack of processability including a high degree of volatile release.

Other examples of known light sensitive compositions include the following. US-A-4,243,743 discloses a composition comprising a poly(amic acid), an organic compound having a photosensitive olefinic double bond and an amino group or a quarternary ammonium salt, and Michler's ketone. US-A-4,321,319 discloses a composition comprising a poly(amic acid) having a photosensitive olefinic double bond, Michler's ketone, nitrofluorene, and 5-nitroacenaphthalene. US-A-4,366,230 discloses a composition comprising a modified poly(amic acid) obtained by introducing a methacryl or acryl group into the ester residue of the poly(amic) acid and Michler's ketone. The compositions disclosed in these three patents produce less than desirable results since a portion of the compositions exposed to light is released when solvent developed and cured.

From EP-A-527 572 benzocyclobutene resin films are known by polymerization of bisbenzocyclobutene monomer in the presence of an unsaturated compound having a higher dienophilic activity than the bisbenzocyclobutene monomer so that an adduct is formed. The polymerization degree of the bisbenzocyclobutene monomer can be controlled by this adduct. The polymerization can be accelerated by UV-irradiation in a nitrogen/oxygen mixed gas atmosphere to give a cured film of 100 % conversion in a shorter time.

It would be desirable to have photocurable polymers with one or more improved physical properties or improved combinations of physical properties, compared to the aforementioned polymers. Some of these properties include a low dielectric constant, a low dissipation factor, low moisture uptake, high sensitivity, high contrast, high resolution, thermal stability, enhanced oxidative stability, resistance to chemicals and plasmas, good adhesion, low release of volatiles, good processability, good planarization, a long shelf life, consistency control, high purity, and low cost.

This object is attained by an a photo-curable, organic-soluble mixture comprising at least one oligomerized cyclobutarene containing 80 wt-% or more of oligomers of a degree of polymerization of three and more of cyclobutarenes of the formula 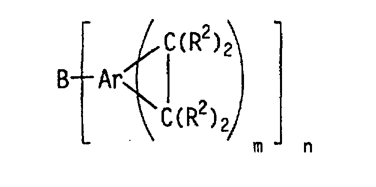 wherein B is a monovalent organic moiety, a direct bond, an n-valent bridging member comprising (1) a polyvalent inorganic moiety, or (2) a polyvalent organic moiety, or B is absent; Ar is a polyvalent aromatic or heteroaromatic moiety, an ar-poly-yl, having three or more valences, provided that two carbon atoms of the cyclobutane ring on the fused side are bonded to adjacent carbon atoms on the same aromatic ring of Ar, n is an integer of one or more and m is an integer of one or more and R² is a monovalent moiety, as its major component;
and at least one photosensitive agent in an amount sufficient to convert the mixture to an organic-insoluble solid upon exposing the mixture to photon radiation.

Preferably 90 wt-% or more of the oligomers have a molecular weight of 1,000 or more as determined from peak area percent by GPC, using a refractive index detector, uncorrected for response factors and as measured against polystyrene standards.

The object is also attained by a photo-curable, organic-soluble mixture comprising at least one oligomerized cyclobutarene as specified above as its major component and at least one photosensitive agent in an amount sufficient to convert the mixture to an organic-insoluble solid upon exposing the mixture to photon radiation.

Organic-soluble means the portion of the nonpoiar mixture not exposed to photons is soluble in hydrocarbons such as Stoddard solvent, xylene, mesitylene, toluene, 2-methoxyethyl ether (diglyme), N-methyl pyrrolidone (NMP), mixtures of NMP and 2-hydroxyethyl 2-pyrrolidone, dipropylene glycol dimethyl ether, n-butyl butyrate or a Stoddard/methanol mixture. When the mixture is polar, organic-soluble means the portion not exposed to photons is soluble in N-methyl pyrrolidone, ethyl lactate, 2-methoxyethyl ether or n-butyl butyrate.

The photo-cured organic-insoluble solid polymer resulting from photon irradiation of the photocurable mixture. These photocured polymers are useful in applications such as photoresists, etching masks, interlayer dielectrics, liquid crystal displays, and flat panel displays.

One embodiment of the invention is directed to a photo-cured pattern-coated substrate having thereon a pattern of a thin film of a mixture of oligomerized cyclobutarenes wherein the coating was exposed to a curing amount of photon radiation so that only a portion of the oligomer was cured and then the film was exposed to solvent development.

Therefore the photocurable mixture is applied to a substrate, portions of the applied mixture are exposed to photon radiation and the substrate is then treated with an organic developing solvent to remove the uncured portion of the mixture; this invention being the pattern-coated substrate resulting from this procedure having the photocurable portion of the mixture adhered thereto.

The photo/thermally cured polymers of this invention have one or more improved physical properties or improved combinations of physical properties, compared to the aforementioned art-known polymers. Such properties may include a low dielectric constant, a low dissipation factor, low moisture uptake, high sensitivity, high contrast, high resolution, thermal stability, enhanced oxidative stability, resistance to chemicals and plasmas, good adhesion, good processability, low release of volatiles, a long shelf life, a high degree of planarization, consistency control, high purity and low cost. These photo/thermally cured polymers are useful in many applications; some of which include composites, laminates, membranes, films, electronics, coatings, and adhesives. The electronic applications include such areas as multichip modules and printed circuit boards.

The cyclobutarenes suitably employed as the major component in the photocurable mixtures of this invention correspond to the formula 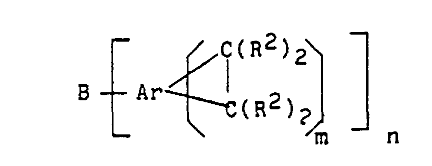 wherein
B is a monovalent organic moiety, a direct bond, an n-valent bridging member comprising (1) a polyvalent inorganic moiety, or (2) a polyvalent organic moiety, or B is absent;
Ar is a polyvalent aromatic or heteroaromatic moiety, an ar-poly-yl, having three or more valences, provided that the two carbon atoms of the cyclobutane ring on the fused side are bonded to adjacent carbon atoms on the same aromatic ring of Ar;
n is an integer of 1 or more;
m is an integer of 1 or more; and
R² is a monovalent moiety.
The synthesis and properties of these cyclobutarenes, as well as the terms used to describe them are found in Gray et al., JACS 100, 2892, (1978); Loon-seng Tan et al., Polymer Preprints, 27(2), 240, (1986); U.S. Patent Application Serial Numbers 07/766,392, 07/701,433, 07/703,957, U.S-A-(s)-4,540,763; 4,783,514 and 4,826,997.

The preferred cyclobutarene monomers correspond to the formulae:

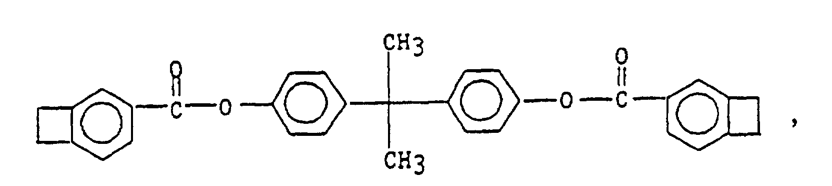

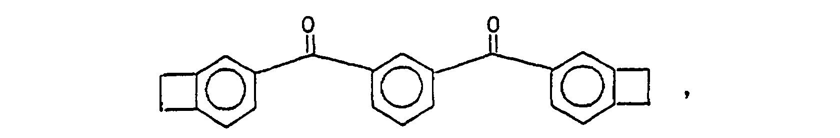

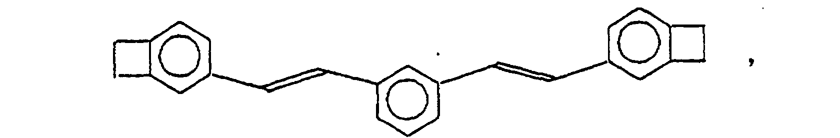

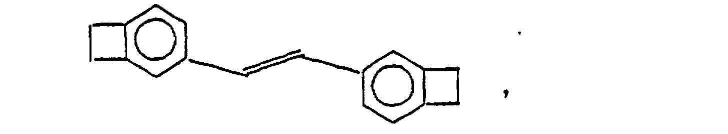

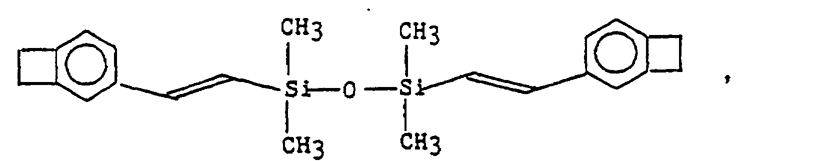

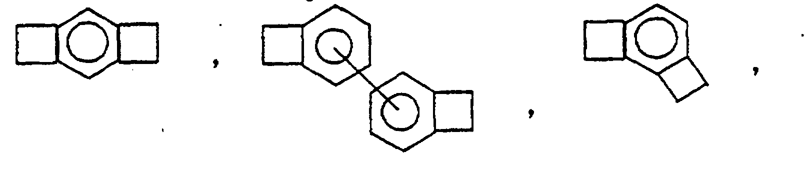

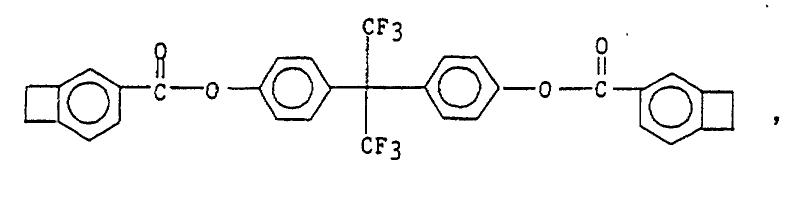

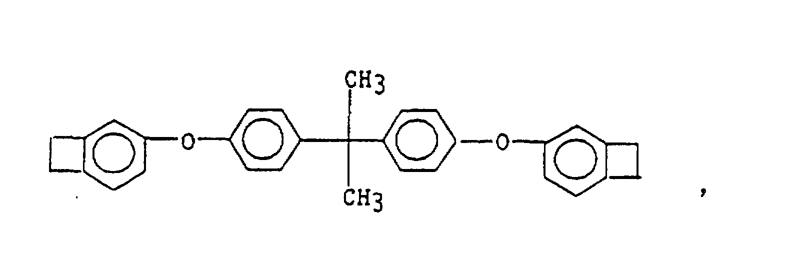

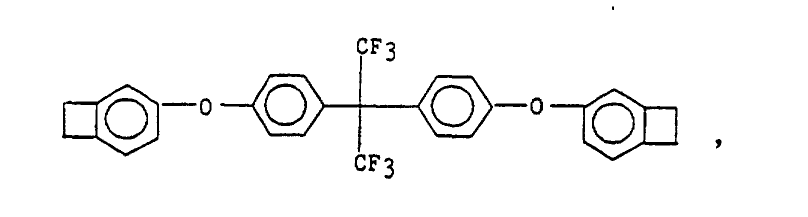

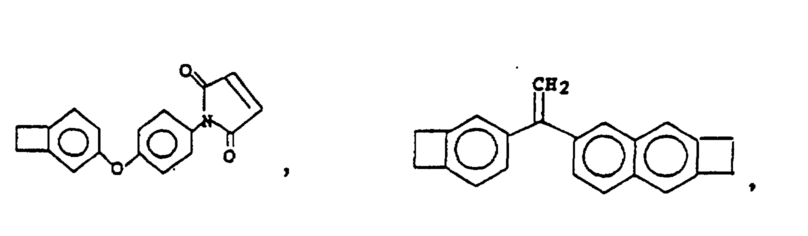

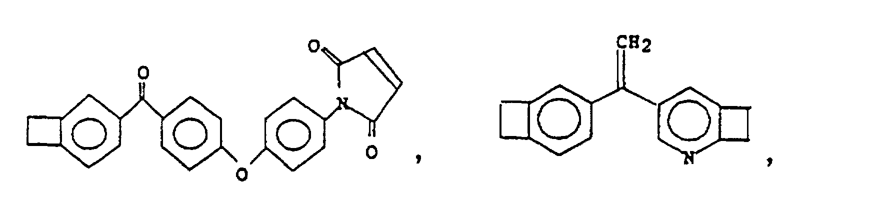

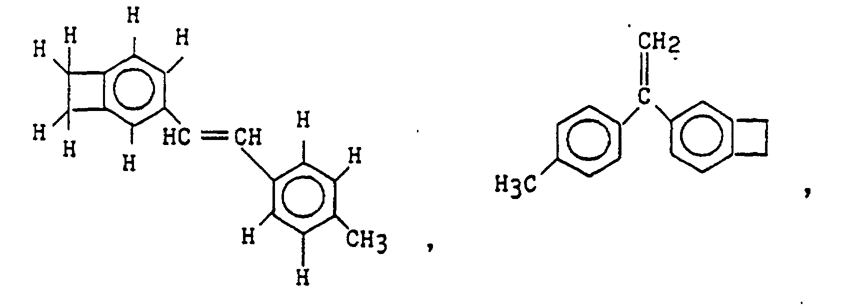

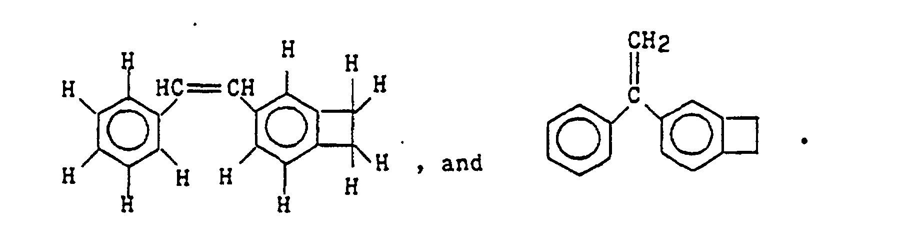

The more preferred cyclobutarene monomers correspond to the formulae: 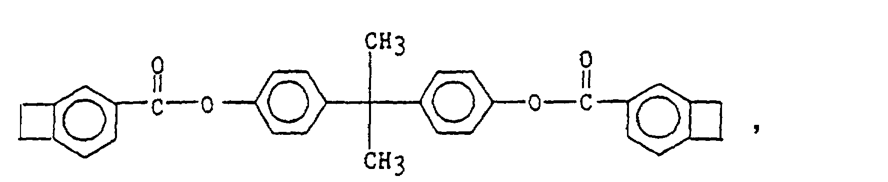 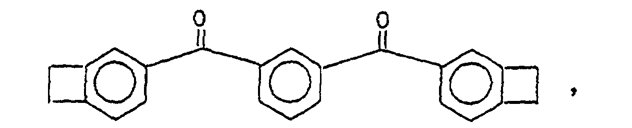 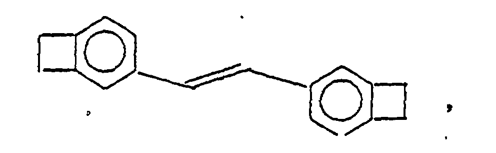 and

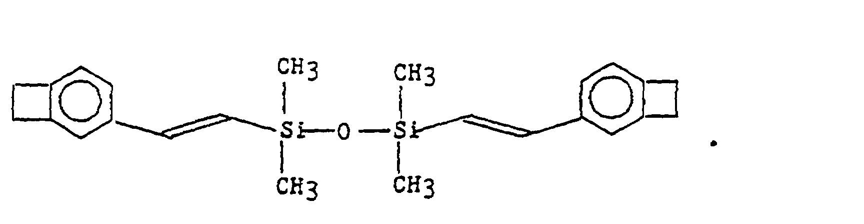

The most preferred cyclobutarene monomer corresponds to the formula:

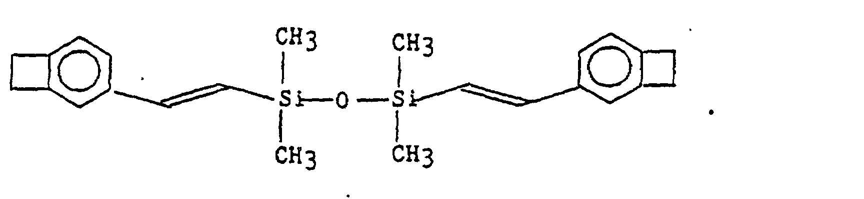

Suitable photosensitive agents employable in this invention are those which have an absorption maximum near the wavelengths of the photon source being used and effect cyclobutarene photocuring. When a photosensitizer is used in conjunction with a photosensitive agent, suitable photosensitive agents are those capable of accepting energy from the photosensitizer.

Preferred photosensitive agents include azides, bismaleimides, acrylates, acetylenes, isocyanates, conjugated aromatic ketones, and benzophenone-containing polymers.

The most preferred group of photosensitive agents is the azides. The azides employed in preparing the polymers of this invention correspond to the formula

Q-(N₃),

wherein
Q is an x-valent organic moiety, and
x is an integer of 1 or more.

Examples of suitable azides, as well as their synthesis and properties are described in "Azides and Nitrenes, Reactivity and Utility", Academic Press, 1984; "Azides and Amines from Grignard Reagents and Tosyl Azide" Smith et al., J. Org. Chem., 34, 3430, (1969); "Encyclopedia of Polymer Science and Engineering", 2nd Edition, Volume 11, 186-212; Yang et al., Proc. SPIE-int. Soc. Opt. Eng., 469(Adv. Resist Technol.), 117-26, 1984; Wolfet al., J. Electrochem. Soc., 131(7), 1664-70, 1984; Tsunoda et al., Photographic Science and Engineering, 17, 390, (1973); Journal of Photographic Science, 29, 188, (1976); "Organic Compounds with Nitrogen-Nitrogen Bonds" Ronald Prez Co., NY, NY, 1966; Boyer et al., Chem. Rev., 54, 1, (1954); Japanese Patent Number J01279240-A, U.S-A-(s) 4,565,767; 4,294,908; 4,354,976, and EP-A(s)-379377, 140319, 119719, 092524.

Preferred azides are aromatic bisazides some examples of which are represented by the following formulae.

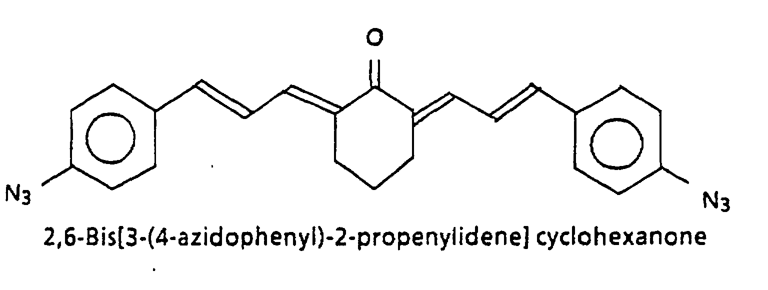

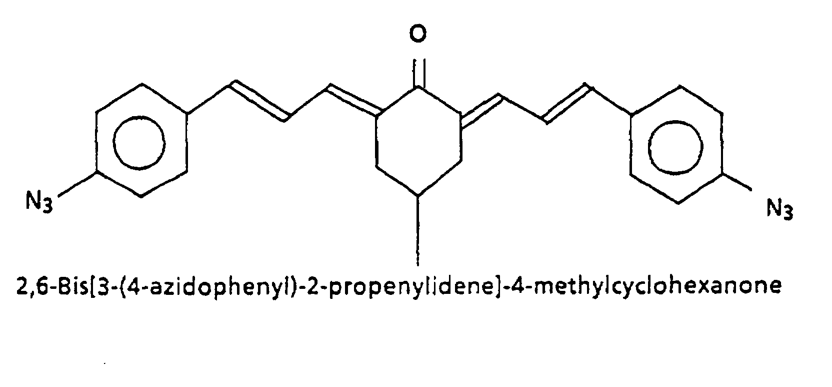

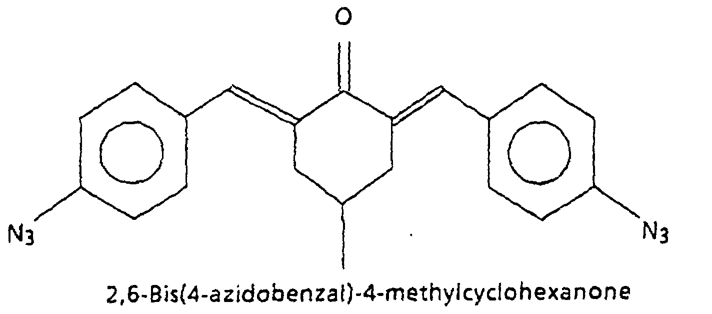

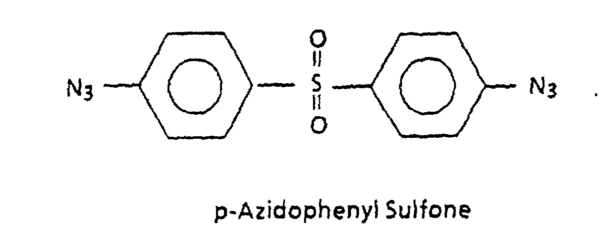

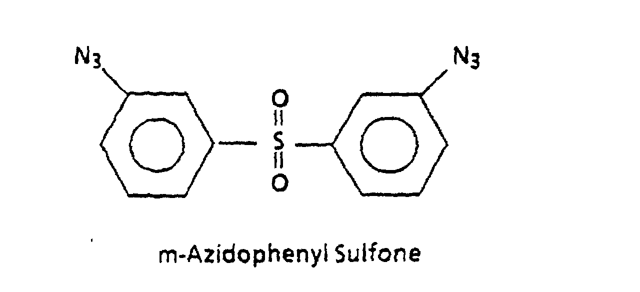

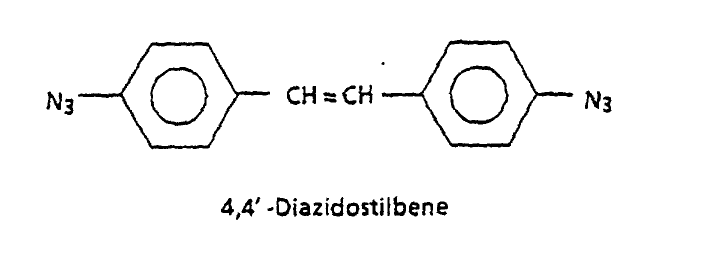

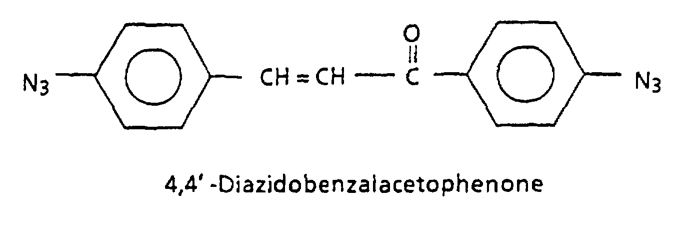

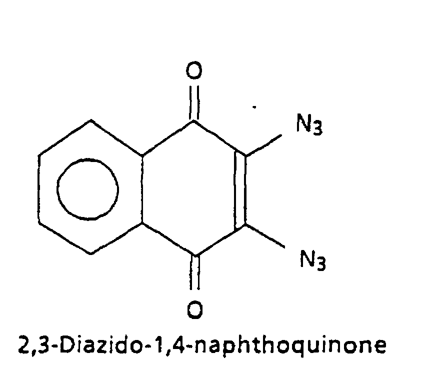

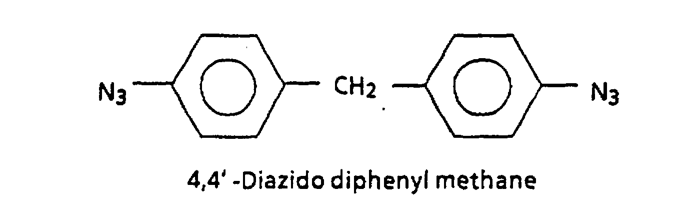

More preferred azides are highly conjugated aromatic bisazides.

The most preferred azide is determined by the wavelengths of the photon source employed. One chooses an azide which has an absorption maximum near the wavelengths of the photon source being used, or if a photosensitizer is being used in conjunction with the photosensitive agent one chooses an azide that will accept energy from the photosensitizer. Solubility of the azide in the system being used is also a consideration.

The amounts of cyclobutarene and photosensitive agent employed in preparing the polymers of this invention can vary. Suitable amounts are those which contain one or more cyclobutarenes as the major component and provide a photocurable mixture from which photocured organic-insoluble polymers can be prepared which have one or more improved physical properties or improved combinations of physical properties, compared to the aforementioned art-known polymers. Such properties include a low dielectric constant, a low dissipation factor, low moisture uptake, high sensitivity, high contrast, high resolution, thermal Stability, enhanced oxidative stability, resistance to chemicals and plasmas, good adhesion, low release of volatiles, good processability, a long shelf life, a high degree of plananzation, consistency control, high purity, and low cost. A suitable amount of photosensitive agent is that which provides sufficient curing in the photon-exposed portion of the mixture to render it insoluble in the developing solvent. A preferred weight percent (hereinafter weight percent) range of photosensitive agent is 0.1 to 20 based on the sum of the weights of the photosensitive agent and the cyclobutarene. A more preferred weight percent range of photosensitive agent is 1 to 6. The most preferred weight percent range of photosensitive agent is 2 to 4. A preferred weight percent range of the cyclobutarene is 80 to 99.9 based on the sum of the weights of the photosensitive agent and the cyclobutarene. A more preferred weight percent range of the cyclobutarene is 94 to 99. The most preferred weight percent range of the cyclobutarene is 96 to 98.

In addition to a cyclobutarene and a photosensitive agent, some embodiments of this invention contain one or more optional components which may be added to tailor the invention's characteristics.

Suitable optional components which may be added to increase the monomer formulation's pot life and the cured resin's toughness include free radical inhibitors such as 2,6-di-tert-butyl-4-methylphenol, tetrakis [methylene (3,5-di-tert-butyl-4--hydroxyhydrocinnamate)] methane, tris (2,4-di-tert-butyl-phenyl)phosphite, thiodiethylene bis-(3,5-di-tert-butyl-4-hydroxy) hydrocinnamate, octadecyl 3,5-di-tert-butyl-4-hydroxy) hydrocinnamate, N,N-diphenyl-p--phenylenediamine, 1,2-dihydro-2,2,4-trimethyldihydroquinoline, triphenylmethane, 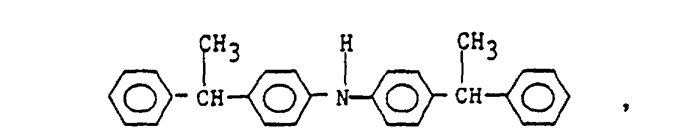 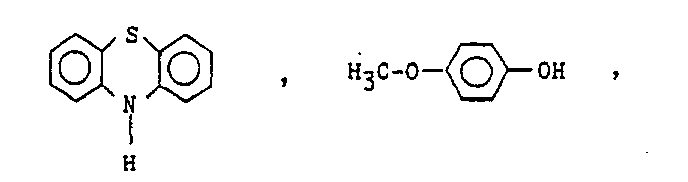 disulfides such as alkylaryl disulfides, dialkyldisulfides, and diaryldisulfides including 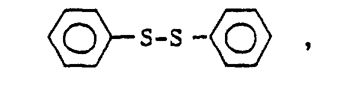 and an isomeric mixture of octylated diphenylamines such as 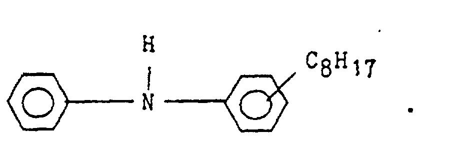 The most preferred free radical inhibitor is 2,6-di-tert-butyl-4-methylphenol.

A suitable amount of the optional free radical inhibitor is that which provides an increased monomer formulation pot life and increased cured resin toughness compared to a formulation without free radical inhibitor. The preferred weight percent range of optional free radical inhibitor is 0 to 10. The more preferred weight percent range of optional free radical inhibitor is 0 to 5, with the most preferred range being 0 to 2.

The monovinylidene mono(cyclobutarene)s that may be added as an optional component to lower the polymerization onset temperature and/or improve the cured polymer's toughness are represented by the following formula 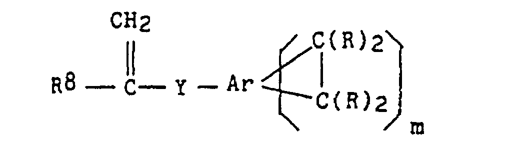 wherein
Ar is a polyvalent aromatic moiety, an ar-poly-yl, having three or more valences, provided that the two carbon atoms on the fused side of the cyclobutane ring are bonded to adjacent carbon atoms on the same aromatic ring of Ar;
Y is a covalent bond or a divalent organic or inorganic moiety;
m is an integer of at least 1;
R is a monovalent moiety; and
R⁸ is a monovalent organic moiety or a heteroatom containing monovalent organic moiety.

The synthesis and properties of these monovinylidene mono(cyclobutarene)s are described in U.S. Patent Application Serial No. 07/766,392.

A suitable amount of the optional monovinylidene mono(cyclobutarene) is that which measurably lowers the polymerization onset temperature and/or improves the cured polymer's toughness. Preferably, the optional monovinylidene mono(cyclobutarene) is employed at a weight percent range of less than 20, more preferably at a weight percent range of less than 15, and most preferably at 1 to 10 weight percent.

Preferred monovinylidene mono(cyclobutarene)s are 1,1-substituted arylethylenes represented by the formula 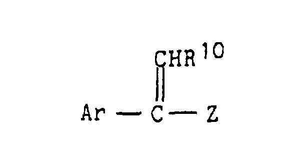 wherein
Ar is a monovalent aromatic or heteroaromatic moiety;
R¹⁰ is H or an alkyl moiety containing 1 to 4 carbon atoms; and
Z is a monovalent aromatic or heteroaromatic moiety, provided that it is a cyclobutarene-containing moiety only when Ar is a cyclobutarene-containing moiety.

The synthesis and properties of these 1,1-substituted arylethylenes are described in U.S. Patent Application Serial No. 07/701,433.

An internal olefinic mono(cyclobutarene) may also be added as an optional component to adjust the cured polymer's crosslinking density and/or improve its toughness. Internal olefinic mono(cyclobutarene) herein refers to a cyclobutarene moiety containing a carbon-carbon double bond wherein neither carbon of the double bond occupies a terminal position of the cyclobutarene moiety. Suitable internal olefinic mono(cyclobutarene)s are represented by the formula 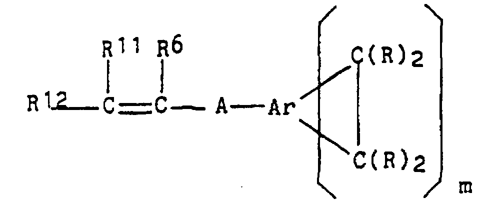 wherein
Ar is a polyvalent aromatic moiety, an ar-poly--yl, having three or more valences, provided that the two carbon atoms on the fused side of the cyclobutane ring are bonded to adjacent carbon atoms on the same aromatic ring of Ar;
A is a covalent bond or a divalent organic or inorganic moiety;
m is an integer of at least 1;
R is a monovalent moiety;
R⁶ and R¹¹ are individually hydrogen or an alkyl moiety; and
R¹² is hydrogen or a monovalent organic moiety provided that when R¹¹ is hydrogen R¹² is a monovalent organic moiety.

The synthesis and properties of these internal olefinic mono(cyclobutarene)s are described in U.S. Patent Application Serial No. 07/766,392.

A suitable amount of optional internal olefinic mono(cyclobutarene) is that which increases the cured polymer's crosslinking and/or improves its toughness. Preferably, the optional internal olefinic mono(cyclobutarene) is employed at a weight percent range of less than 20, more preferably at a weight percent range of less than 15, and most preferably at 1 to 10 weight percent.

Maleimidocyclobutarenes which may be added as an optional component to increase the cured polymer's toughness are represented by the formula 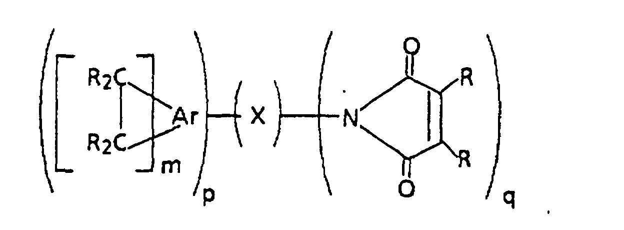 wherein
X is a direct bond or a divalent organic or inorganic moiety;
Ar is a polyvalent aromatic moiety, an ar-poly--yl, having three or more valences, provided that the two carbon atoms on the fused side of the cyclobutane ring are bonded to adjacent carbon atoms on the same aromatic ring of Ar;
m, q, and p are integers of 1 or more; and
R is a monovalent moiety.

The synthesis and properties of these maleimidocyclobutarenes are described in U.S-A-4,783,514 and 4,826,997.

A suitable amount of the optional maleimidocyclobutarene is that which increases the cured polymer's toughness. Preferably, the optional maleimidocyclobutarene is employed at a weight percent range of less than 20, more preferably at a weight percent range of less than 15, and most preferably at 1 to 10 weight percent.

The optional polymaleimide monomers which may be added to increase the cured polymer's thermal stability and/or improve its toughness correspond to the formula 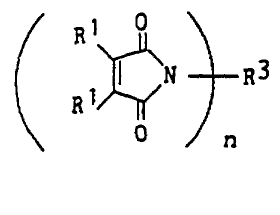 wherein
n is an integer of 2 or greater;
R¹ is separately and independently in each occurrence, a monovalent moiety, provided that R¹ does not interfere with polymerization; and
R³ is an n-valent bridging member comprising an n-valent organic moiety or heteroatom-containing organic moiety.

The synthesis and properties of these optional polymaleimide monomers are described in U.S. Patent Application Serial No. 07/630,902.

A suitable amount of the optional polymaleimide monomer is that which increases the cured polymer's toughness and/or thermal stability. Preferably, the optional polymaleimide is employed at a weight percent range of less than 20, more preferably at a weight percent range of less than 15, and most preferably at 1 to 10 weight percent.

An antioxidant may be added to increase the formulation's oxidative stability during processing as well as in the cured resin. Antioxidants of the phenol-, sulfide-, phosphite-, and amine type may be employed in this invention. Hindered amines are the preferred antioxidants. Hindered amines with aliphatic and aromatic moieties are more preferred antioxidants. The most preferred antioxidant is polymerized 1,2-dihydro-2,2,4--trimethylquinoline, CAS registry number 26780-96-1.

Preferably, the optional anti oxidant is employed at a weight percent range of less than 8, more preferably at a weight percent range of less than 7, and most preferably at .001 to 6 weight percent.

A photosensitizer may be added to increase the photosensitive agent's photosensitivity. The synthesis and properties of suitable optional sensitizers are disclosed in Specht et al., Tetrahedron, Vol. 38, No. 9, p 1203-1211, (1982); Tsunoda et al., Photographic Science and Engineering, 17,390,(1973); US-A-4,268,603; and EP-A-379377. Suitable photosensitizers are those whose absorption maximum is near the wavelengths of the photon source employed. Preferred photosensitizers are represented by the following formulae. 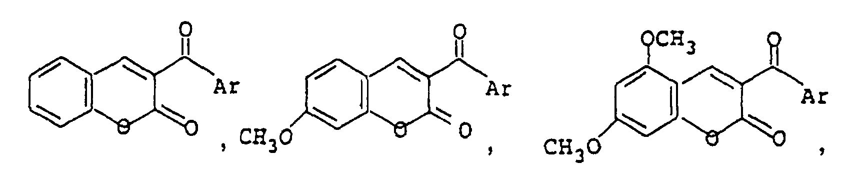 where Ar is represented by the following formulae: 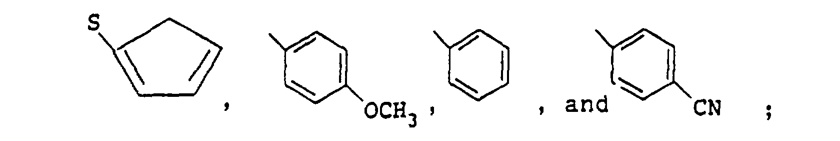 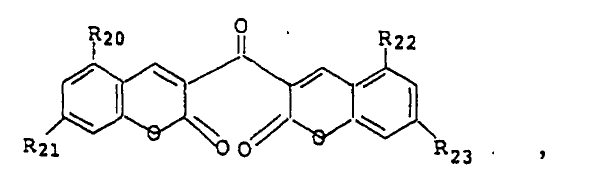 where R²⁰, R²¹, R²², R²³ are separately and independantly H, OCH₃, and -N(C₂H₅)₂; 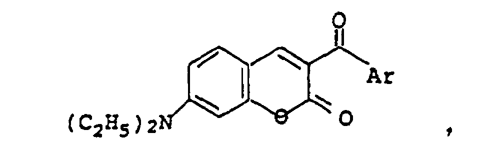 where Ar is represented by the following formulae: 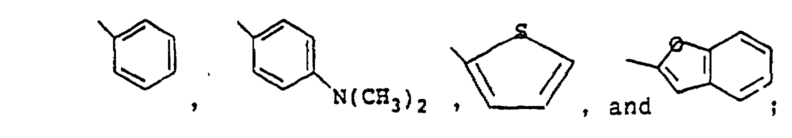 and 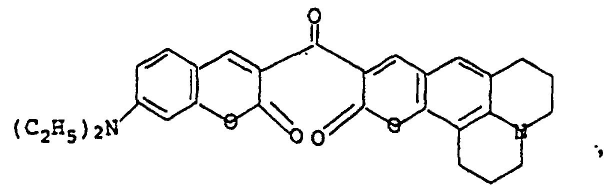 and

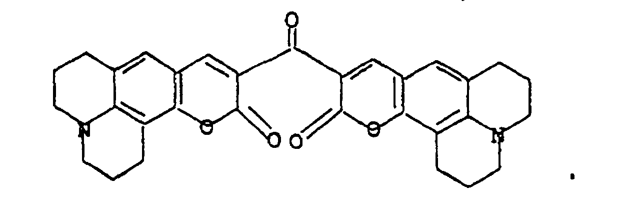

More preferred photosensitizers are represented by the following formulae. 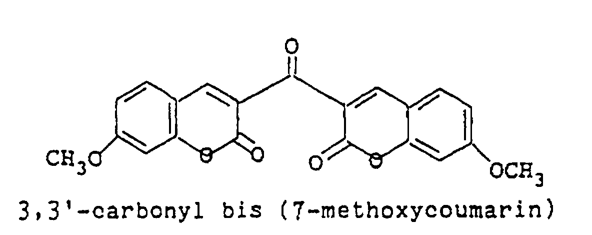 and

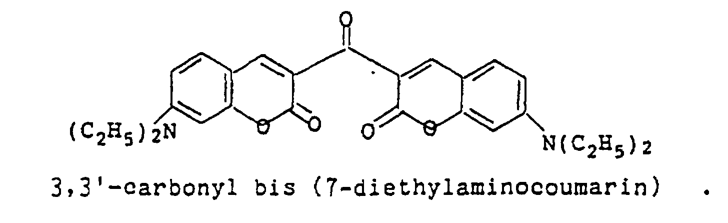

Preferably, the optional photosensitizer is employed at a weight percent range of less than 5, more preferably at a weight percent range of less than 3, and most preferably at .001 to 2 weight percent.

Preferably the cyclobutarene-containing portion of the formulation and any optional components are oligomerized or B-staged prior to use to improve handling, processing, and performance characteristics. The cyclobutarene may also be oligomerized without any photosensitive agent or other optional components present. The molecular weight of a resin directly impacts its performance in a photoresist system. It is most preferred to have the highest molecular weight possible while maintaining a high solubility level. It is further desirable to have low polydispersity (Mw/Mn; where Mw is the weight-average molecular weight and Mn is the number-average molecular weight) so that the soluble oligomers are easily converted into an insoluble gel when exposed to a photon source. This will result in the maximum solubility difference between the exposed and unexposed areas of the polymer film covering the wafer or substrate. Mw can be controlled by the extent of conversion during oligomerization or B-staging. With the most preferred cyclobutarene, divinyltetramethyl-disiloxane bisbenzocyclobutane, represented by the formula 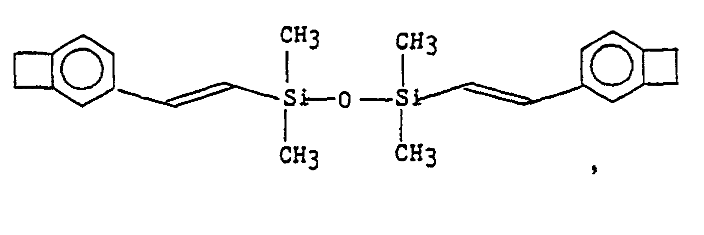 the gel point occurs while a significant portion of monomer and low molecular weight oligomers are present due to the complex nature of the linking group in this poly(cyclobutarene). The extent of conversion also affects the molecular weight distribution.

The average molecular weight and molecular weight distribution may also be altered by selectively removing some oligomeric mixture components. A wider range of oligomer molecular weights results in a wider range of oligomer solubilities after photocuring. The lower molecular weight materials remain soluble after photocuring, thus it is desirable to remove them from the formulation prior to coating it on a substrate to minimize losses upon solvent development. The lower molecular weight materials may also be removed from the oligomerized cyclobutarene prior to adding a photosensitizer or other optional components. Preferably at least 80 weight percent of the cyclobutarene monomer is removed prior to coating the solution on a substrate. More preferably at least 80 weight percent of the cyclobutarene monomer and at least weight 50 percent of the cyclobutarene dimer is removed prior to coating the solution on a substrate. Even more preferably at least 80 weight percent of the cyclobutarene monomer and dimer is removed prior to coating. Most preferably at least 90 weight percent of the cyclobutarene monomer and dimer and at least 50 weight percent of the cyclobutarene trimer are removed.

The preferred oligomer may contain 80 weight percent or more of oligomers of a degree of polymerization of three or more. The more preferred oligomer may contain 90 weight percent or more of oligomers of a degree of polymerization of three or more. The most preferred oligomer may contain 95 weight percent or more of oligomers of a degree of polymerization of three or more. By a degree of polymerization of three or more is meant species of the molecular weight of trimers and higher.

One may determine the percentage of monomer, dimer and trimer in the oligomerized cyclobutarene by gel permeation chromotography (GPC). The weight percentage of monomer, dimer and trimer are approximately equal to the peak areas of the GPC trace. They are not exact because detection is by refractive index and the response factors for the monomer and dimer may vary somewhat from that of the oligomer. The approximation may be used and is used in the data in this specification. For more accurate determinations, the response factors for the monomer and dimer should be determined.

A preferred cyclobutarene oligomer may contain 80 weight percent or more of oligomers of a molecular weight of 1,000 or more as determined from peak area percent by GPC, using a refractive index detector, uncorrected for response factors and as measured against polystyrene standards. A more preferred cyclobutarene oligomer may contain 90 weight percent or more as determined from peak area percent of oligomers of a molecular weight of 1,000 or more, similarly determined. A most preferred cyclobutarene oligomer may contain 95 weight percent or more of oligomers of a molecular weight of 1,000 or more, similarly determined.

Various methods may be used to obtain the preferred molecular weight distribution of the cyclobutarene oligomer. The desired cyclobutarene monomer may be oligomerized or B-staged neat by heating. Then the lower molecular weight fractions may be removed by extraction in a nonsolvent for the higher molecular weight species. For example, one may dissolve the cyclobutarene oligomer in a miscible solvent and then mix it with a nonsolvent to precipitate out the higher molecular weight species. For the DVS-BCB, mesitylene may be used as the miscible solvent and an alcohol such as t-amyl alcohol may be used as the precipitating solvent.

One means of effecting this removal is alcoholic precipitation, as exemplified in Example 16. Other techniques commonly used to separate the fractions of a polymer mixture may also be employed here, such as chromatography and solvent extraction, including supercritical solvent extraction.

B-staging of benzocyclobutanes is described in US-A-4,642,329. When neat B-staging is complete, a solvent, sometimes called a casting solvent, is used to dissolve the B-staged material and thus may be used to facilitate its removal from the B-staging apparatus. Hydrocarbons are suitable solvents for most nonpolar oligomeric cyclobutarene-containing systems. Preferred solvents for most nonpolar oligomeric cyclobutarene-containing systems include xylene and mesitylene. The most preferred solvent is mesitylene. B-staging may also be performed in solution.

Suitable solvents for polar oligomeric cyclobutarene-containing systems include N-methyl pyrrolidone, ethyl lactate, 2-methoxyethylether, 2-methoxyethylether, and gamma butyrolactone. The most preferred solvent for polar oligomeric cyclobutarene-containing systems is N-methyl pyrrolidone.

Some systems require a smalt quantity of cosolvent to solubilize the photosensitive agent when it is present at the high end of its specified weight percent range. When the photosensitive agent is an azide, suitable cosolvents include ethers, glycol ethers, ketones, and esters. Preferred cosolvents are 2-methoxyethanol, 2-ethoxyethanol, 2-methoxyethylether, and 2-ethoxyethylether. The most preferred cosolvent is 2-ethoxyethylether.

The photosensitivc agent can be dissolved in the B-staged resin/solvent system by heating in an oven, water bath, or by sonication. The most preferred method is a water bath. All manipulations of the cyclobutarene/photosensitive agent mixture are preferably performed in a darkened environment to prevent premature initiation of the photosensitive reaction by photon radiation. One means of providing a suitable environment is by using working space equipped with amber filtered (yellow) lights.

Thin films of the cyclobutarene-containing formulation may be applied to substrates without the use of an adhesion promoter. When desirable, an optional adhesion promoter is formulated as a spray- or spin-on solution which is applied immediately after solvent cleaning of the substrate and immediately before applying the cyclobutarene-containing formulation. Alternatively, the adhesion promoter is added to the cyclobutarene/photocrosslinking agent formulation. The adhesion promoter is designed such that one end of the motecule either covalently attaches or adsorbs to the metal, metal oxide, or ceramic substrate surface, while the second end of the molecule reacts or interacts with the cyciobutarene polymer matrix. Suitable adhesion promoters include trialkoxyvinylsilanes and trialkoxyvinylsilyl benzocyclobutanes. More preferred adhesion promoters include include 3-aminopropyltriethoxysilane, trimethoxyvinylsilane, triethoxyvinylsilane, trimethoxyvinylsilyl benzocyclobuianes, and triethoxyvinylsilyl benzocyclobutanes. The most preferred adhesion promoter is 3-aminopropyltriethoxysilane. The preparation and properties of trialkoxyvinylsilyl benzocyclobutanes are described in US.-A-(s) 4,831,172 and 5,002,808.

Suitable substrates are comprised of silicon, alumina, and a variety of ceramic materials such as aluminum nitride. More preferred substrates are comprised of alumina and silicon. The most preferred substrate is comprised of silicon.

The cyclobutarene-containing formulations are applied from solutions containing 10-70 weight percent solids. Solids content and the molecular weight of the cyclobutarene-containing formulation determine the viscosity of the spray- or spin-on solution. Spin time and speed are used to control film quality and thickness at a particular formulation viscosity. Details of substrate coating with benzocyclobutane films can be found in the Journal of Electronic Materials, Vol 19, No. 12, 1990.

The majority of the casting solvent is removed during the spin coating process. A softbake cycle may be required to remove residual solvent. The softbake also relaxes stress resulting from flow of the polymer film, increases the film's adhesion to the substrate, and hardens the film for more convenient handling during processing; for example, to prevent adhesion to a mask when printing in a hard contact mode. A preferred softbake temperature is one sufficient to remove residual solvent, provide stress relaxation which requires a temperature above the polymer's glass transition temperature, but low enough to avoid oxidizing or thermal curing of the resin: The preferred softbake temperature will vary depending in-part on the components of the cyclobutarene-containing formulation. A more preferred softbake temperature ranges from 80°C to 150°C. The most preferred softbake temperature ranges from 80°C to 120°C. When using a photosensitizer it is most preferred to use 80°C as the softbake temperature because of the thermal instability of the photosensitizer.

A preferred softbake time is one sufficient to remove residual solvent, provide stress relaxation, but short enough to avoid oxidizing or thermal curing of the formulation components. The preferred softbake time will vary depending in part on the components of the cyclobutarene-containing formulation. A more preferred softbake time ranges from 15 seconds to 60 minutes. The most preferred softbake time range depends on balancing desired performance results with maximizing throughput, may vary from 15 seconds to 30 minutes. To maximize throughput, the minimum time would be optimal.

Suitable softbake atmospheres include a vacuum, air, nitrogen, argon, and helium. Nitrogen is the most preferred atmosphere.

The manner in which the films are cooled affect their performance. The slower the cooling, the denser the vitrified polymer. A high density, low void volume polymer is resistant to solvent penetration. A decreased solvent penetration rate results in a reduced resist development rate.

Exposure dose is dependent upon the photon source being used. An increase in the photon source intensity may help to overcome high optical density problems associated with thick polymeric films upto 30µm(micrometers). Selective removal of various components of a high pressure mercury photon source may provide superior film performance. Suitable photon sources include those for which a suitable photosensitive agent exists that can absorb that photon source's wavelengths of energy. Preferred photon sources include visible light, ultraviolet light, X-rays, and electron beams. More preferred photon sources include ultraviolet and visible light. The most preferred photon source is dependent upon the photosensitive agent being used and should be chosen such that its photon emissions are near the absorption maximum of the photosensitive agent or photosensitizer employed.

Following photon exposure, an optional softbake cycle may be employed. This cycle increases the reaction rate of long-lived photochemically generated intermediates. These intermediates have increased mobility during this cycle and thus may migrate and find a reactant species.

An alternative means of increasing the mobility of these reactive intermediates is heating during photon exposure. Such a procedure increases the photosensitive agent's sensitivity.

Once photon exposure is complete, the film is solvent developed. Solvent development comprises removing, using a solubilizing solvent, the material that has not been exposed to photon radiation and thus not photo-cured. Dissolution involves two steps. The first is solvent penetration which converts the glassy polymer to a swollen network. The second step involves extracting low molecular weight oligomers from the gel at the solution interface.

Preferred solvent development methods include spray or immersion techniques. Spray development is the more preferred technique. Suitable developing solvents are those which selectively dissolve the nonphoton-exposed film component. Suitable solvents for nonpolar polymer film systems include hydrocarbons such as Stoddard solvent, xylene, mesitylene and toluene, 2-methoxyethyl ether (diglyme), n-butyl butyrate, dipropylene glycol dimethyl ether, N-methyl pyrrolidone (NMP), mixtures of NMP and 2-hydroxyethyl 2-pyrollidone and a Stoddard/methanol mixture. Stoddard solvent as used herein is defined at page 1095, "Hawley's Condensed Chemical Dictionary", 11th Edition, Van Nostrand Reinhold Company, New York, 1987. The most preferred solvent for nonpolar polymer film systems is n-butyl butyrate.

Suitable developing solvents for polar polymer film systems include N-methyl pyrrolidone, mixtures of n-butyl butyrate and ethyl lactate, 2-methoxyethyl ether (diglyme), n-butyl butyrate, dipropylene glycol dimethyl ether, N-methyl pyrrolidone (NMP) and mixtures of NMP and 2-hydroxyethyl 2-pyrollidone. The most preferred solvent for polar polymer film systems is n-butyl butyrate.

One property that makes polymeric insulators useful in multichip module fabrication is their ability to planarize topographical features. The definition obtainable in photoresists is limited by the wavelength of the photon source employed and thus thickness variations can be detrimental to the quality of the module produced. For this reason it is important to have good planarization.

At this point in the process, the patterned thin film may have additional microcircuitry and photodefined dielectric layers applied to it or it can be further thermally cured. Procedures for preparing multilayer interconnect units or multichip modules are disclosed in the following references: J.J. Reche, "Fabrication of High Density Multichip Modules", IEEE/CMT 1989 IEMT Symposium, p.104; T. Tessier et al., "Process Considerations in Fabricating Thin Film MCM's", IEPS 1989, p.294; S.F. Hahn et al., "The Fabrication and Properties of Thermoset Films Derived from Bisbenzocyclobutene for Multilayer Applications", Proceedings of the ACS Division of Polymeric Materials: Science and Engineering, 59, 190, 1988; P.H. Townsend et al., "The Processing and Properties of Multilayer Interconnection Structures Using Thermoset Films Derived From Bisbenzocyclobutene", Proceedings of the Materials Research Society, p.47, 1989; J. Reche et al., "High Density Multichip Module Fabrication", The International Journal for Hybrid Microelectronics, Vol. 13, No.4, 1990. Additional information on preparing multichip modules may be found in "Benzocyclobutene Processing Guide For Multilayer Interconnect Fabrication (Multichip Modules)", The Dow Chemical Company, Midland, Michigan, 1991.

The aforementioned photo-cured polymers are then subjected to heat for sufficient time to complete the curing process. The following preferred heating cycle may be employed for further curing in a nitrogen atmosphere.
50°C for 0.5 hour
50°C to 250°C over 1 hour
250°C for 1 hour
250°C to 100°C over 2 hours

Preferably, cyclobutarenes are cured in an atmosphere of 100 ppm or less of oxygen to minimize oxidation.

The following examples are illustrative only, and do not limit the scope of the invention. All percents stated in the following examples are by weight unless otherwise noted. All weight percents stated are relative to the percent of the combined weight of polyazide and cyclobutarene present in the system, unless otherwise noted.

### Example 1 - Preparation of Photosensitive, Patterned Thin Films From 2,6-bis(4-azidobenzylidene)-4-Methylcyclohexanone and Oligomeric Divinyltetramethyldisiloxane Bisbenzocyclobutane

A sufficient quantity of a 2,6-bis(4-azidobenzylidene)-4--methylcyclohexanone(BAC-M) (347.3 mg, 0.938 mmol) was added to a darkened vial containing oligomeric divinyltetramethyldisiloxane bisbenzocyclobutane (DVS-BCB) (20.0636g, 55 percent solids in mesitylene, 11.035 g polymer, Mw = 31000, Mn = 1300) to form a 3 percent solution of BAC-M. The vial was wrapped in aluminum foil and heated to 60°C for 45 minutes to dissolve the BAC-M. The DVS-BCB/BAC-M mixture was transferred to an Eberbach mixer and shaken for 40 minutes.

The DVS-BCB/BAC-M solution was transferred to a class 1000 cleanroom for deposition on bare silicon wafers or substrates. Prior to deposition, the wafers were cleaned using a xylene stream applied to the wafer during a spin cycle. A stream of adhesion promoter, a 0.5 percent solution of triethoxyvinylsilylbenzocyclo-butane(TES-BCB) in water, was applied to the wafer for 2 seconds during a subsequent spin cycle using a syringe connected to a 0.2 µm filter, afterwhich the wafer was spun dry.

Applying the DVS-BCB/BAC-M solution to the substrate begins by pouring it onto the center of the stationary silicon substrate, which was then followed by a spin coating step. The spin coating procedure employed for the DVS-BCB/BAC-M solution, using a Solitec Model 5100 spin coater, involveed two steps and produced a 5 µm coating after thermal cure. The first step was a spread cycle during which the substrate or wafer was spun at 500 rpm for 3 seconds to completely cover it with resin. The second step involved accelerating the substrate to 5000 rpm and holding it at that speed for 30 seconds.

The film close to the edge of the wafer, often called the edge bead, comprised concentric polymer rings that were somewhat thicker than the remainder of the film. The edge bead was removed using a xylene stream during a spin cycle. Edge bead removal increased the film thickness uniformity and made manipulating the substrate easier.

Residual solvent not removed during the spin coating process was removed using a softbake cycle in an oven containing a nitrogen atmosphere at 80°C for 0.5 hours. After cooling to ambient temperature, the substrate was exposed for 5 minutes using a USHIO 250 watt medium-pressure mercury-xenon lamp contained within a Canon PLA501FA mask aligner through a patterned chrome-on-glass plated mask in the contact printing mode. The contact printing mode as used herein refered to direct contact between the mask and the film. This mode produced the highest resolution possible.

Solvent development, which was the dissolution and removal of film areas not exposed to photons, was effected by swirling the substrate while it was immersed in Stoddard solvent for 1 minute. Development produced the negative image in the film of the pattern contained on the mask which included 10 x 10 µm vias.

The substrate was thermally cured under a nitrogen atmosphere using the following cure cycle.
50°C to 100°C over 5 minutes
100°C for 15 minutes
100°C to 150°C over 15 minutes
150°C for 15 minutes
150°C to 250°C over 1 hour
250°C for 1 hour
250°C to 100°C over 2 hours

The step height of the film as measured by profilometry, using a Tencor instruments Alpha-Step 200 computerized surface profiler, was 2.48 µm, which was 48 percent of the control film thickness. The stylus mass used was 5 mg, the scan length 400 µm, and the scan rate 5 µm/second.

### Example 2 - Effect of Exposure Dose on Residual Film Thickness

A 3 percent solution of BAC-M in DVS-BCB resin, prepared according to the procedure described in Example 1, was coated on a series of bare silicon wafers using the procedure described in Example 1. The substrates were exposed to the photon source of Example 1 for 1, 2, 3, 5,10, and 15 minutes. The photon-exposed coated wafers were solvent developed using two immersion cycles of 30 seconds in Stoddard solvent. After a hard cure using the procedure described in Example 1, the film thickness was measured by stylus profilometry. Table 1 shows the effect of exposure dose on residual film thickness.

**Table 1**

| Effect of Exposure Dose on Residual Film Thickness | | |
|---|---|---|
| Exposure Time (Minutes) | Exposure dose (mJ/cm²) | % Residual Film Thickness |
| 1 | 232.8 | 29.8 |
| 2 | 465.6 | 33.3 |
| 3 | 698.4 | 35.6 |
| 5 | 931.2 | 47.8 |
| 10 | 1164.0 | 46.5 |
| 15 | 3492 | 52.5 |

This data indicated that film thickness was influenced by exposure dose. Exposure time was a function of the photon source and preferably was minimized to increase throughput. The minimum exposure time was that necessary for the photons to penetrate the film sufficiently to cure the bottom layer of the film in contact with the substrate. Crosslinking at this interface prevented delamination of the polymer from the substrate.

### Example 3 - Effect of Development Solvent Contact Time on Residual Film Thickness

A 3 percent solution of BAC-M in DVS-BCB resin, prepared according to the procedure described in Example 1, was coated on a series of bare silicon wafers using the procedure described in Example 1. The films were exposed to the photon source for 15 minutes to ensure completion of the photochemical reaction. The wafers were quartered and one section was not developed thus serving as a control for the initial film thickness. The wafers were immersed in Stoddard sol vent for different time periods. The films were dried under a nitrogen stream after development and thermally hard cured using the procedure described in Example 1. The film thickness of each sample, as measured by stylus profilometry, is shown in Table 2.

**Table 2**

| Film Thickness as a Function of Development time | | |
|---|---|---|
| Development Time(s) | Film Thickness (µm) | % Residual |
| 0 | 5.16 | 100 |
| 10 | 3.345 | 64.8 |
| 20 | 2.925 | 56.7 |
| 30 | 2.895 | 56.0 |
| 45 | 2.875 | 55.6 |
| 60 | 2.630 | 50.9 |
| 90 | 2.84 | 54.6 |
| 120 | 2.63 | 50.6 |
| 180 | 2.66 | 51.2 |

Tabie 2 showed that the majority of film thickness losses occur in the first 20 seconds of solvent development. This characteristic allowed for a wide range of development times with little effect on the film thickness.

### Example 4- Effect of Photosensitive Agent Concentration on Residual Film Thickness

One, two, three, and four percent solutions of BAC-M in DVS-BCB resin were coated on a series of bare silicon wafers using the procedure described in Example 1. The films were solvent developed for 1 minute in Stoddard solvent followed by hard curing. The film thicknesses differ as shown in Table 3.

**Table 3**

| Variation in Film Thickness with % BAC-M | |
|---|---|
| % BAC-M | Film Thickness (µm) |
| 1 | 0 |
| 2 | 1.572 |
| 3 | 2.035 |
| 4 | 2.948 |

The data shows that film thickness can be increased by increasing the percent BAC-M.

### Example 5 - Effect of Cosolvent on Normalized Film Thickness

BAC-M (315.4 mg, 0.852 mmol, 5.4 percent) was added to a darkened vial containing oligomeric DVS-BCB (9.9974 g, 55 percent solids in mesitylene, 5.498 g polymer, Mw = 27100, Mn = 1300) in a darkened laboratory. A 0.5353 g portion of 2-methoxyethanol was added to dilute the polymer to 52.2 percent solids based on the sum of the weights of polymer and solvents. After shaking on an Eberbach mixer for five minutes at a high setting, the solution was heated at 60°C for 0.5 hours. The sample was shaken on the high setting for 0.5 hours as it is cooled to ambient temperature. The agitation and heating cycles were repeated followed by a 5 hour shake at a low setting, afterwhich the BAC-M was fully solubilized.

Additional solutions are prepared at 6.3 percent BAC-M(370.4 mg), and 7.3 percent BAC-M(435.4 mg) using 0.5247 and 0.5314 g of 2-methoxyethanol, respectively. The 6.3 percent sample was almost completely soluble and the 7.3 percent sample even less soluble. Additional methoxyethanol, 0.2858 g and 0.5010 g, was added to the 6.3 percent and 7.3 percent BAC-M samples, respectively. After shaking on an Eberbach mixer for 5 minutes on the high setting, the solutions were heated at 60°C for 0.5 hours. The samples were shaken for 0.5 hours on a high setting as they were cooled to ambient temperature. This procedure completely solubilizes the 6.3 percent sample, but not the 7.3 percent sample.

Film preparation was performed as described in Example 1, except that solvent development was performed by spraying the wafer with solvent thereby forming a puddle of solvent on the wafer. The wafer was stationary for 5 seconds followed by the spread and spin cycles of Example 1. The samples were thermally hard cured using the procedure described in Example 1. The film thickness of each sample, as measured by stylus profilometry, is shown in Table 4.

**Table 4**

| Normalized Film Thickness as a Function of % BAC-M | |
|---|---|
| %BAC-M | Normalized Film Thickness |
| 1 | 7.3 |
| 2 | 27.3 |
| 3 | 37.1 |
| 4 | 50.2 |
| 5 | 64.4 |
| 6 | 64.1 |
| 7 | 70.8 |

This data showed that the normalized (compared to a control for each percent BAC-M solution) film thickness increased significantly with increased in the percent BAC-M. Using 2-methoxyethanol permits BAC-M dissolution at 5 and 6 weight percent in a mesitylene solution containing polymer at 55 percent solids. Such levels were not reproducibly attainable using mesitylene alone.

### Example 6 - Effect of resin Molecular Weight on Film Retention

A 27,100 weight-average molecular weight DVS-BCB resin was subjected to the same film preparation, exposure, solvent development, and analysis as described in Example 1. The thermal cure cycle is as follows.
50°C for 0.5 hour
50°C to 250°C over hour
250°C for 1hour
250°C to 100°C over 2 hours
The residual film thickness of this system was 36.3 percent of the control film thickness.

A 44,000 weight-average molecular weight DVS-BCB resin (Mn = 1360) was prepared by increasing the extent of conversion by B-staging for a longer time period. It was diluted to 55 percent solids to allow a valid film retention comparison to the previously described film system comprising a 27,100 molecular weight polymer. This was done by diluting with mesitylene and shaking for 1 hour using an Eberbach mixer on the high setting prior to adding the 3 percent BAC-M. The light sensitive formulation was prepared by the procedure described in Example 1. The residual film thickness of this system was 48.3 percent of the control film thickness.

A 73,000 weight-average molecular weight DVS-BCB resin (Mn = 1400) was prepared by further increasing the extent of conversion by B-staging. This material surpasses its gel point. The gels are removed by filtration. It was diluted to 55 percent solids with mesitylene and shaken for 1 hour using an Eberbach mixer on the high setting prior to adding the 3 percent BAC-M photosensitive agent. The 3 percent BAC-M was added under amber filtered yellow lights to prevent premature photocuring of the mixture. After dissolving the BAC-M, the mixture was taken to a class 1000 cleanroom having filtered amber lights and was subjected to the same coating, softbake, exposure, solvent development, and hard cure as the two previous examples. Surface profilometry indicated the resultant film retained 58.2 percent of the initial film thickness.

These three examples demonstrate that film retention was increased by increasing the cyclobutarene resin molecular weight.

### Example 7 - Effect of Varying the Oligomer Percent Solids Content

The 73,000 molecular weight DVS-BCB resin from Example 6 was used at 60 percent solids, in the following protocol. BAC-M (377.7 mg, 1.02 mmol, 3.0 weight percent) was added to a darkened vial containing oligomeric DVS-BCB (20.0022 g, 60 percent solids in mesitylene, 12.0013 g polymer, Mn = 1,400, Mw = 73,000) in a darkened laboratory. The vial was wrapped in aluminum foil and heated for 45 minutes in an oven at 60°C. The sample was removed from the oven and shaken on an Eberbach mixer at a high setting for 20 minutes as it cooled to ambient temperature. After treatment with spread and spin cycles and softbake conditions as described in Example 1, the wafer was exposed to the photon source described in Example 1 for 15 minutes through a mask. The wafer was quartered and one section not developed thereby serving as a reference for the initial film thickness. One section is immersion developed for 30 seconds in Stoddard solvent. All of the films are thermally hard cured as described in Example 6.

The film's masked areas that are solvent developed with Stoddard solvent are 8.4 µm thick as measured by stylus profilometry, which translates to a 27.9 percent film loss relative to the undeveloped portion which measures 11.55 µm. This film retention of 72.1 percent at 60 percent solids can be compared to the 58.2 percent film retention of the 55 percent solids 73,000 molecular weight system of Example 6.

This data indicates that higher polymer solids loadings in mesitylene produces thicker polymer coatings. At excessive solids loadings, fibers form which emanate over the edge of the wafer which can complicate processing.

The aspect ratio of this film system, as measured by electron microscopy, is approximately 0.5 for a 9.1 µm high line separated by 18.2 µm from its nearest neighboring feature.

### Example 8 - Effect of the DVS-BCB Molecular Weight Distribution on Film Retention

The oligomeric DVS-BCB of Example 5 (40.0 g, 55 percent solids in mesitylene, 22.0 g polymer, Mn = 1300, Mw = 27100) was placed in an addition funnel positioned high enough above a blender of rapidly stirring isopropanol to prevent oligomer precipitation prior to transferring the oligomer mixture to the blender. The oligomer mixture was added dropwise to the blender being operated at a high shear rate over a 75 minute period. A white precipitate was formed which was removed from the blender using a spatula and filtered using a medium porosity fritted funnel and a water aspirator as a vacuum source. The precipitate was dried at 50°C for 8.5 hours in a vacuum oven attached to a vacuum source of less than 30 inches of Hg. The resulting white amorphous glassy material was pulverized using a mortar and pestle thus producing 20.1 g (91.4 percent recovery) of a nontacky white powder. Size exclusion chromatography indicated that the molecular weight distribution (Mn = 1,700, Mw = 31,000) was changed such that the monomer content was reduced from 12.9 percent to 7.4 percent and the dimer content reduced from 10.7 percent to 9.7 percent. Film retention in this system was 42.7 percent following photon exposure and solvent development procedures described in Example 1. This was in comparison to the film retention of 36.3 percent for the polymer system of Example 6 wherein there was no change of the molecular weight distribution.

### Example 9 - Comparison of Spray and immersion Development

A series of DVS-BCB oligomeric solutions containing 1-4 percent BAC-M were prepared as described in Example 4. One group was spray developed while the second was immersion developed. The results shown in Table 5 indicate that the two procedures produce similar film thicknesses.

Spray development was effected by spraying a stream of Stoddard solvent onto the center of the wafer during spin and spread cycles of 33 seconds followed by a spin cycle to dry the wafers.

immersion development was effected by swirling the wafers in Stoddard solvent for 60 seconds as described in Example 1 followed by drying the wafers under a nitrogen stream.

**Table 5**

| Film Thickness as a Function of Development Method | | |
|---|---|---|
| %BAC-M | Thickness(µm) Spray | Thickness(µm) Immersion |
| 1 | 0 | 0 |
| 2 | 1.69 | 1.57 |
| 3 | 2.16 | 2.04 |
| 4 | 2.95 | 2.66 |

### Example 10 - Effect of Varying The Developing Solution

A 3.0 percent solution of BAC-M(426.3 mg) in oligomeric DVS-BCB(24.9700 g, 55 percent solids in mesitylene, 13 7335 g polymer) was prepared by the procedure described in Example 5. Films were prepared from this solution according to the procedure described in Example 1 with the exception that the adhesion promoter in this system was triethoxyvinyl silane in acetic acid which was prepared as a 0.5 percent solution in deionized water. This solution was aged for a minimum of 15 minutes prior to use and can be used for a maximum of 8 hours after preparation. After a thermal cure according to the procedure of Example 6, the film thickness was measured by stylus profilometry relative to a control wafer for each of the developing solutions. The results are shown in Table 6.

**Table 6**

| Film Thickness as a Function of Developing Solution | | |
|---|---|---|
| Developer | Thickness (µm) | % Retention |
| Stoddard solvent | 1.76 | 33.1 |
| Mesitylene | 1.92 | 35.6 |
| Xylene | 1.68 | 31.2 |

This data indicates that a variety of materials can be used for solvent development of these films. Optimization of the developing solution was possible by adding nonsolvents such as methanol or isopropanol as a means of slowing the film removal rate, thereby retaining a greater percentage of the initial film thickness.

### Example 11 - Preparation of Photosensitive, Patterned Thin Films From 2,6-bis(4-azidobenzylidene)-4-Methylcyclohexanone and Oligomeric 3,3'-(ethenyl)bis-bicyclo(4.2.0)octa-1,3,5-triene

A 60 percent solids solution of oligomerized 3,3'-(ethenyl)bis-bicyclo(4.2.0)octa-1,3,5-triene(9.9722 g total, 5.9833 g polymer, Mn = 600, Mw = 2000) in xylene was combined with 420 mg of 2,6-bis(4-azidobenzylidene)-4-methylcyclohexanone(BAC-M) under amber lights The solution was shaken for 20 minutes on an Eberbach mixer set on its highest speed. The solution was then heated to 60°C and held at that temperature for 30 minutes thereby producing a homogeneous solution. After cooling to ambient temperature over 30 minutes, additional BAC-M (608.7 mg total, 9.2 percent) was added to the solution which was then heated for an additional 30 minutes at 60°C The resulting solution, containing some insoluble material, was coated onto substrates using the procedure described in Example 1. The film coated substrates were heated in a nitrogen atmosphere to 120°C and maintained at that temperature for 30 minutes. After a 5 minute cool down period they were immersion-developed for 1 minute in mesitylene. The resulting film coated substrates were cured using the thermal cure cycle described in Example 6. Profilometry results indicate that the developed films have step heights of 4.148 µm while the photon-exposed, undeveloped control films have a thickness of 4.147 µm.

### Example 12 - Preparation of Photosensitive, Patterned Thin Films from 2,6-bis(4-azidobenzylidene)-4-methylcyclohexanone and an Eqimolar Mixture of Oligomeric 3,3'-(ethenyl)bis-bicyclo(4.2.0)octa-1,3,5-triene and Divinyltetramethyldisiloxane Bisbenzocyclobutane

A 55 percent solids solution of oligomerized 3,3'-(ethenyl)bis-bicyclo(4.2.0)octa-1,3,5-triene/divinyltetramethyldisiloxane bisbenzocyclobutane copolymer(EL-BCB/DVS-BCB)(9.1922 g total, 5.0557 g polymer) in xylene was combined with 402.5 mg of 2,6-bis(4-azidobenzylidene)-4-methylcyclohexanone(BAC-M) under amber lights. The solution was shaken for 20 minutes on an Eberbach mixer set on its highest speed. The solution was then heated to 60°C and held at that temperature for 30 minutes thereby producing a homogeneous solution. After cool ing to ambient temperature over 30 minutes, additional BAC-M (515.8 mg total, 113.3 mg BAC-M, 9.3 percent) was added to the solution which was then heated for an additional 30 minutes at 60°C. The resulting solution, containing some insoluble material, was coated onto substrates using the procedure described in Example 1. The film coated substrates were heated in a nitrogen atmosphere to 120°C and maintained at that temperature for 30 minutes. After a 5 minute cool down period, they were immersion developed for 1 minute in Stoddard solution. The resulting film coated substrates were cured using the hard cure cycle described in Example 6. Profilometry results indicate that the developed films have step heights of 4.135 µm while the photon-exposed, undeveloped control films have a thickness of 5.432 µm.

### Example 13 - Preparation of Photosensitive, Patterned Thin Films from 2,6-bis(4-azidobenzylidene)-4-methyicyclohexanone, Oligomeric Divinyltetramethyldisiloxane Bisbenzocyclobutane and Polymerized 1,2-dihydro-2,2,4-trimethylquinoline

Under amber filtered lights BAC-M(174.4 mg, 0.47 mmol, 3.0 weight percent) was added to an amber vial containing a 1 percent solution of polymerized 1,2-dihydro-2,2,4-trimethylquinoline (PDTQ) in oligomeric DVS-BCB (10.2128 g, 55 percent solution in mesitylene, 5.5614 g polymer, 55.6 mg PDTQ). The mixture was dissolved and processed by the procedure described in Example 10. Stoddard solvent was used as the immersion developer for 1 minute.

A second mixture was prepared using an antioxidant comprising PDTQ modified by aromatization or hydrogenation(PDTQ-M). BAC-M(159.9 mg, 0.43 mmol, 3.1 weight percent) was added to an amber vial containing a 1 percent solution of PDTQ-M in oligomenc DVS-BCB (9.2418 g, 55 percent solution in mesitylene, 5.0327 g polymer, 50.3 mg PDTQ-M). The mixture was processed according to the procedure described for PDTQ. The profilometry results for these films are shown in Table 8.

**Table 8**

| Antioxidant Effect on % Film Retention | | | |
|---|---|---|---|
| Antioxidant | Film Thickness (µm) | Control Film Thickness(µm) | % Retention |
| PDTQ | 1.414 | 4.886 | 28.9 |
| PDTQ-M | 1.639 | 5.612 | 29.2 |

This data suggests that antioxidants do not drastically affect the film retention of photodefinable mixtures.

### Example 14 - Effect of Photosensitizer 3,3'-Carbonyl bis(7-diethylaminocoumarin)

Meta-azidophenyl sulfone (0.4331 g, 300.30 g/mol, 1.44 mmol, 3.0 weight percent) and 3,3'-carbonyl bis(7-diethylaminocoumarin) (0.0730 g, 460.53, 0.16 mmol) were added under amber filtered lights to an amber vial containing a solution of oligomeric DVS-BCB (25.3257g, 55 percent solids in mesitylene, 13.9291 g polymer). The vial was capped and heated in a water bath at 60°C for 1 hour. The bisazide was not completely soluble at this point. The mixture was heated at 60°C for an additional 1/2 hour. The sample was shaken for 1 hour using an Eberbach shaker on a high setting resulting in a homogeneous solution.

A 4 inch silicon wafer was spin coated with the solution at a spin speed of 5,000 rpms for 30 seconds. The wafer was softbaked at 80°C for 1/2 hour under nitrogen. Prior to exposure, the wafer was cooled to room temperature. It was exposed to 350 to 450 nm UV light for 15 seconds using a 1,000 watt high pressure mercury-xenon arc lamp, and then immersion-developed for 2 minutes using Stoddard solvent. The wafer was then thermally cured (Tₘₐₓ = 250°C). After solvent development and cure, profilometry results indicate a film thickness of 4.1 microns.

A UV-Vis spectrum of a DVS-BCB solution from 300 nm to 500 nm at a scan rate of 60 nm per minute was very similar to that obtained for a DVS-BCB/bisazide mixture, thus indicating that this bisazide absorbed in the same region as the DV5-BCB, which was at lower waveiength than desired.

The significance of these results was that the bisazide (meta-azidophenyl sulfone)/DVS-BCB mixture without photosensitizer was not curable by exposure to 350 to 450 nm UV light, however, when 0.5 percent of photosensitizer was added the sensitivity of the mixture increases significantly.

Very similar results were obtained when the 3,3'-carbonyl bis(7-diethylaminocoumarin) photosensitizer was replaced with 3,3'-carbonyl bis(7-dimethoxycoumarin).

### Example 15 - Effect OF Precipitating Oligomeric DVS-BCB in Refluxing Alcoholic Solvents

Oligomeric DVS-BCB (40.5 g, 60 percent solids in mesitylene, 24.3 g polymer, Mw = 44000, Mn = 1360) was placed in a pressure-equalizing addition funnel connected to a claisen adapter which was inserted into a 3-necked 1000 mL resin kettle containing 700 mL of isopropanol. The resin kettle was equipped with an immersion well to control the solution temperature and a reflux condenser, equipped with a nitrogen outlet, which was connected to the portion of the claisen adapter not directly above the stirring solution. The resin kettle was purged with nitrogen for 15 minutes prior to heating and continuously purged at a slow rate thereafter. The isopropanol was brought to reflux prior to the addition process. The oligomer was added dropwise over a period of 113 minutes to the solution which was stirred at 300 rpm using a mechanical stirrer equipped with teflon blades at both the bottom and top of the isopropanol solution. After addition was completed, the mixture was stirred for an additional 15 minutes prior to removing the stirring and heating sources. After sitting for 16 hours overnight, the apparatus was disassembled and the mixture filtered. The solids were washed with three 50 mL portions of isopropanol and removed using a spatula. The resin was pulverized using a mortar and pestle to increase the surface area of the solid to facilitate drying. The resin was dried in a vacuum oven at 50°C for 2 hours and then at 90°C for 18 hours to remove any residual isopropanol and consolidate the resin. The resin collapses indicating that the polymer's glass transition temperature was 90°C or above. The high molecular weight material recovered weighed 16.4 g (67.5 percent).

The soluble components of this separation were concentrated using a rotary evaporator giving 8.1 g of viscous resin for later characterization by size exclusion chromatography. The overall material balance was 16.4 + 8.1 g = 24.5 g (101 percent) indicating that very little isopropanol was left in the low molecular weight component.

The resulting change in the molecular weight distribution was from Mn = 1300, Mw = 40000 to Mn = 4100, Mw = 57000. The polymer was redissolved in mesitylene at 55 percent solids after heating at 60°C for 1 hour. A 3 weight percent BAC-M formulation was prepared and films produced therefrom by spin coating at 5000 rpm. After immersion development for 1 minute in Stoddard solvent and cure, the film thickness of the patterned film was 7.2 µm (66 percent film retention) with a control film thickness of 10.9 µm compared to the 5 µm coating produced from 55 percent solutions of the unaltered material.

Using polymer precipitated in isopropanol results in a nearly complete removal of the monomeric and dimeric components of the resin. However, the film loss of approximately 33 percent described above indicated that higher oligomeric materials were responsible for some loss. Therefore, in an effort to remove a greater quantity of the higher molecular weight oligomers, high boiling alcohols are used producing the data in Table 9.

**Table 9**

| Polymer Precipitation Results: Characterization of Precipitated B-staged DVS-BCB | | | | | | | |
|---|---|---|---|---|---|---|---|
| Alcohol | b.p. °C | Dielectric Constant | Mn | Mw | Monomer (%) | Dimer (%) | (%) Recovery |
| NONE | | | 1300 | 38500 | 12.2 | 10.6 | NA |
| Isopropyl | 82.3 | 18.3 | 4100 | 57000 | 1.3 | 2.2 | 67.5 |
| t-butyl | 83 | 10.9 | 5700 | 74000 | 1.2 | 1.7 | 51.8 |
| 2-butyl | 99-100 | 15.8 | 5300 | 73000 | 1.2 | 1.8 | 58.4 |
| 1-pentyl | 136-138 | 13.9 | 6100 | 72000 | 1.5 | 1.6 | 47.7 |
| 2-pentyl | 119-120 | 14.2 | 6600 | 71000 | 1.1 | 1.4 | 51.0 |
| 3-methyl | 130 | 14.7 | 5900 | 87000 | 1.2 | 1.6 | 53.9 |
| 1-butyl | | | | | | | |
| 3-methyl | 112 | | 7100 | 83000 | 1.0 | 1.4 | 50.4 |
| 2-butyl | | | | | | | |
| 2-methyl | 102 | 5.82 | 8100 | 99000 | 1.2 | 1.4 | 41.1 |
| 2-butyl | | | | | | | |
| 2-methyl | 130 | | 6400 | 67000 | 1.0 | 1.5 | 53.1 |
| 1-butyl | | | | | | | |

The most preferred alcohol for precipitating the higher molecular weight oiigomeric components of DV5-BCB is t-amyl alcohol (2-methyl 2-butyl), which removes a portion the DVS-BCB trimer.

Removing the low molecular weight components results in both greater film retention and greater film thickness at the same solids concentration.

### Example 16 - Effect of Solvent Precipitated DVS-BCB Resin Molecular Weight on Percent Film Retention

A 57,000 weight average molecular weight DVS-BCB resin was prepared by the same preparation method as described in Example 15 using isopropyl alcohol as the extraction solvent and was subjected to the same film preparation, solvent development, and analysis as described in Example 1. 2-methoxyethyl ether was used as a development solvent for a development time of 90 seconds and the wafers were given an exposure dose of 1,000 mJ/cm² at the 365 nm wavelength. The films were spun at a spin speed of 5,000 rpms after a 10 second spread cycle at 500 rpms. The thermal cure cycle was as follows:
50°C for 5 minutes
50°C to 100°C over 5 minutes
100°C for 15 minutes
100°C to 150°C over 15 minutes
150°C for 15 minutes
150°C to 250°C over 60 minutes
250°C for 60 minutes
250°C to 100° over 120 minutes

The residual film thickness of this system was 58.8 percent of the control film thickness after development and 60.8 percent after cure.

In effort to maximize the film retention after development and cure, other high molecular weight resins that were prepared as described in Example 15 using various alcohols for precipitation. The resins were evaluated producing the data in Table 10.

**Table 10**

| Percent Film Retention of Precipitated B-staged DVS-BCB Resins | | | | |
|---|---|---|---|---|
| Precipitating Alcohol | Mw | Mn | Film Thickness After Development (µm) (%Retention) | Film Thickness After Cure (µm) (% Retention) |
| isopropyl | 57,000 | 4,100 | 2.62 (58.8) | 2.72 (60.9) |
| 2-methyl,1-butanol | 67,000 | 6,400 | 3.89 (72.3) | 3.97 (73.8) |
| 2-pentanol | 71,000 | 6,600 | 4.34 (75.5) | 4.42 (77.0) |
| 1-pentanol | 72,000 | 6,100 | 4.32 (78.0) | 4.41 (79.7) |
| 2-butanol | 73,000 | 5,300 | 3.14 (61.8) | 3.33 (65.6) |
| 3-methyl,2-butanol | 83,000 | 7,100 | 4.57 (79.1) | 4.65 (80.5) |
| 3-methyl,1-butanol | 87,000 | 5,900 | 4.00 (71.6) | 4.14 (74.2) |
| t-amyl | 99,000 | 8,100 | 5.51 (86.6) | 5.57 (87.5) |
| t-amyl | 128,000 | 4,900 | 5.63 (87.4) | 5.76 (89.4) |

The most preferred resin for obtaining films with good film retention after development and cure is the resin precipitated in t-amyl alcohol (2-methyl, 2-butyl alcohol) which has a film retention of 86.6 percent after development and 87.5 percent after cure.

The higher the weight average and number average molecular weight of the DVS-BCB resin the greater the film retention after development and cure.

### Example 17 - Effect of Film Thickness on Film Retention

Oligomeric DVS-bis-BCB (567.6 g, 56 percent solids in mesitylene, 317.8 g solids was poured into a pressure equalizing addition funnel. The addition funnel was connected to a 5L resin kettle with a bottom drain. T-amyl alcohol (3.5 L, Aldrich, 99percent) was added to the 5L reaction flask. The reactor was purged with nitrogen slowly throughout the remainder of the procedure. The alcohol was heated to reflux (bp. 102°C) and the polymer solution was added. After the addition was completed, the reactor cooled to 35°C over a period of approximately 3 hours. The homogeneous polymer solution became cloudy at 71°C. A white high density fraction settled to the bottom of the reactor. Subsequently, the viscous white resin was decanted through the stopcock. After the decantation was completed, the material was dried inside a vacuum oven at 110°C for 15 h producing 129.8 g (40.8percent) of a white polymer.

The procedure was repeated nine additional times and the resultant polymer mixed together to give a masterbatch of material with the following molecular weight distribution: Mn 4800, Mw 95,000 and Mz 295,000.

A 3 percent BAC-M formulation was prepared as described in Example 1. Prior to polymer deposition, an adhesion promoter, 3-aminopropyltriethoxysilane (3-APS), was applied as a 0.5 percent aqueous solution (w/w) at 5000 rpm

The resin was spin coated on 100 mm silicon wafers using a spread cycle at 500 rpm for 10 seconds in which the polymer solution flowed out to cover the substrate followed by a final spin speed (variable see Table 11) which was maintained for 30 seconds. After exposure (1000 mJ/cm² at 365 nm wavelength), the films were developed using an appropriate solvent. The film thickness was measured after development and after the final cure for developed films. The control film thickness was measured after cure which was preceded by exposure.

**Table 11:**

| Film Thickness for 3 wt percent BAC-M/DVS-bis-BCB Solutions (10 second spread time) | | | | | |
|---|---|---|---|---|---|
| % Solids | RPM Spin Speed | Film Thickness (µm) Control | Film Thickness (µm) After Dev. | Film Thickness (µm) After Cure | Percent Retention |
| Developed in Stoddard Solvent | | | | | |
| 45 | 2000 | 9.253 | 9.685 | 8.319 | 89.9 |
| | 3500 | 6.914 | 6.185 | 5.895 | 85.3 |
| | 5000 | 5.718 | 5.420 | 4.940 | 86.4 |

| Developed in Diglyme | | | | | |
|---|---|---|---|---|---|
| 45 | 2000 | 9.285 | 8.085 | 8.110 | 87.3 |
| | 3500 | 6.870 | 5.800 | 5.835 | 84.9 |
| | 5000 | 5.700 | 4.870 | 4.895 | 85.9 |

### Example 18: Thicker Films by Multiple Coatings and Exposures Followed by one Solvent Development Step

In an attempt to obtain thicker photopatterned polymer coatings, a multiple photopolymer coating and exposure process was developed.

1.006 grams of an oligomer of 1,2-dihydro-2,2,4-trimethylquinoline available as AgeRite MA was added to 199.358 grams of a 50 percent solids DVS-bis-BCB resin (molecular weight of Mn = 4,500 g/mole; Mw = 59,000 g/mole) solution in mesitylene and dissolved with heating at 60°C in a water bath shaker for 1 hour. 3.085 g of BAC-M was added to the solution and dissolved with heating at 60°C in a water bath shaker for 30 minutes. The solution was then filtered through a 0.5 micron filter.

A clean silicon wafer with patterned aluminum metal deposited on top of the thermal silicon oxide, was spin coated with a 0.5 percent aqueous solution of 3-aminopropyltriethoxysilane adhesion promoter at a spin speed of 2,500 rpms for 30 seconds. The photodefinable solution was then spin coated using a 10 second spread cycle of 500 rpms followed by a spin speed of 2,500 rpms for 30 seconds to yield a 8.0 micron thick film. The wafer was placed in a nitrogen purged oven at 80°C for 30 minutes. After cooling to room temperature, the wafer was placed in an Oriel mask aligner and the aluminum pattern on the wafer was aligned to the photomask. The photodefinable DVS-bis-BCB film on the wafer was then given an exposure dose of 700 mJ/cm² of light measured at the 365 nm wavelength (broad band source 350-450 nm). A second photodefinable DVS-bis-BCB film was spin coated on the first exposed film at a spin speed of 7,500 rpms to give a film coating thickness of 8.0 microns. The wafer was placed in a nitrogen purged oven at 80°C for 30 minutes. After cooling to room temperature, the wafer was placed in an Oriel mask aligner and the aluminum pattern on the wafer was aligned to the photomask. The second photodefinable film was given an expose dose of 700 mJ/cm² measured at the 365 nm wavelength (broad band source 350-450 nm). The wafer was then immersed in 2-methoxy ethyl ether for 90 seconds and sprayed dry with a nitrogen stream. The wafer was placed in a nitrogen purged oven at 80°C for 10 minutes to further dry. The total film thickness for the photodefinable DVS-bis-BCB was measured using a profilometer by draging the profilometer stylus over a via hole patterned into the film. The film thickness after development was approximately 12.23 microns. The film was cured according to the following cure schedule:
50°C for 5 minutes
50°C to 100°C over 5 minutes
100°C for 15 minutes
100°C to 150°C over 15 minutes
150°C for 15 minutes
150°C to 250°C over 60 minutes
250°C for 60 minutes
250°C to 100°C over 120 minutes
The cured polymer film was continuous and of high quality with a film thickness of 12.93 microns.

### Example 19 Thicker films by use of a DVS-bis-BCB formulation with a mixture of bisazides

In an attempt to decrease the optical density and maintain high film retention after development and cure a DVS-bis-BCB formulation with a mixture of bisazides was developed.

0.134 grams of m-azidophenyl sulfone (m-sulfone) was added to 24.040 grams of a 55.6 percent solids DVS-bis-BCB resin (molecular weight of Mn = 4,500 g/mole; Mw = 59,000 g/mole) solution in mesitylene and dissolved with heating at 60°C in a water bath shaker for 30 minutes. The solution was then placed in the sonicator for 30 minutes and then back into a water bath shaker at 60°C for 30 minutes to help dissolve the m-azidophenyl sulfone. 0.269 grams of BAC-M was added to the solution and dissolved with heating at 60°C in a water bath shaker for 30 minutes. The solution was then placed in the sonicator for 20 minutes and filtered through a 5.0 micron filter into a clean amber bottle.

0.266 grams of BAC-M was added to 23.767 grams of a 55.6 percent solids DVS-bis-BCB resin (molecular weight of Mn = 45,00 g/mole; Mw = 59,000 g/mole) solution in mesitylene and dissolved with heating at 60°C in a water bath shaker for 30 minutes. The solution was then placed in the sonicator for 20 minutes. Both solutions were formulated at the same time.

A clean silicon wafer was spin coated with a 0.5 percent aqueous solution of 3-aminopropyltriethoxysilane adhesion promoter at a spin speed of 3,000 rpms of 30 seconds. The 1 percent m-azidophenyl sulfone/2percent BAC-M solution was then spin coated on the wafer using a 10 second spread cycle of 500 rpm followed by a spin speed of 3,000 rpm for 30 seconds to yield a 10.83 micron thick film. The wafer was placed in a nitrogen purged oven at 80°C for 30 minutes. After cooling to room temperature, the wafer was placed in an Oriel Mask aligner and the photodefinable DVS-bis-BCB film on the wafer was then given an exposure dose of 1,000 ml/cm² of light, measured at the 365 nm wavelength, (broad band source 350-450 nm) through a quartz photomask. The film thickness was measured with a profilometer to be 10.83. The wafer was then immersed in 2-methoxy ethyl ether for 90 seconds and sprayed dry with a nitrogen stream. The wafer was placed in a nitrogen purged oven at 80C for 10 minutes to further dry. After solvent development and drying, the film thickness was 9.22 microns as measured by a profilometer. The film was cured according to the following cure schedule:
50°C for 5 minutes
50°C to 100°C over 5 minutes
100°C for 15 minutes
100°C to 150°C over 15 minutes
150°C for 15 minutes
150°C to 250°C over 60 minutes
250°C for 60 minutes
250°C to 100°C over 120 minutes
The film thickness after cure was 9.450 microns as determined by a profilometer.

In an effort to maximize the film retention and film thickness after development and cure, other wafers were spin coated with the 1 percent m-azidophenyl sulfone/2percent BAC-M solution at various spin speeds. For comparative purposes, the solution with 2 percent BAC-M was also evaluated at the same spin coating conditions. The resins were evaluated for film thickness after each processing step as described above producing the data in Table 12.

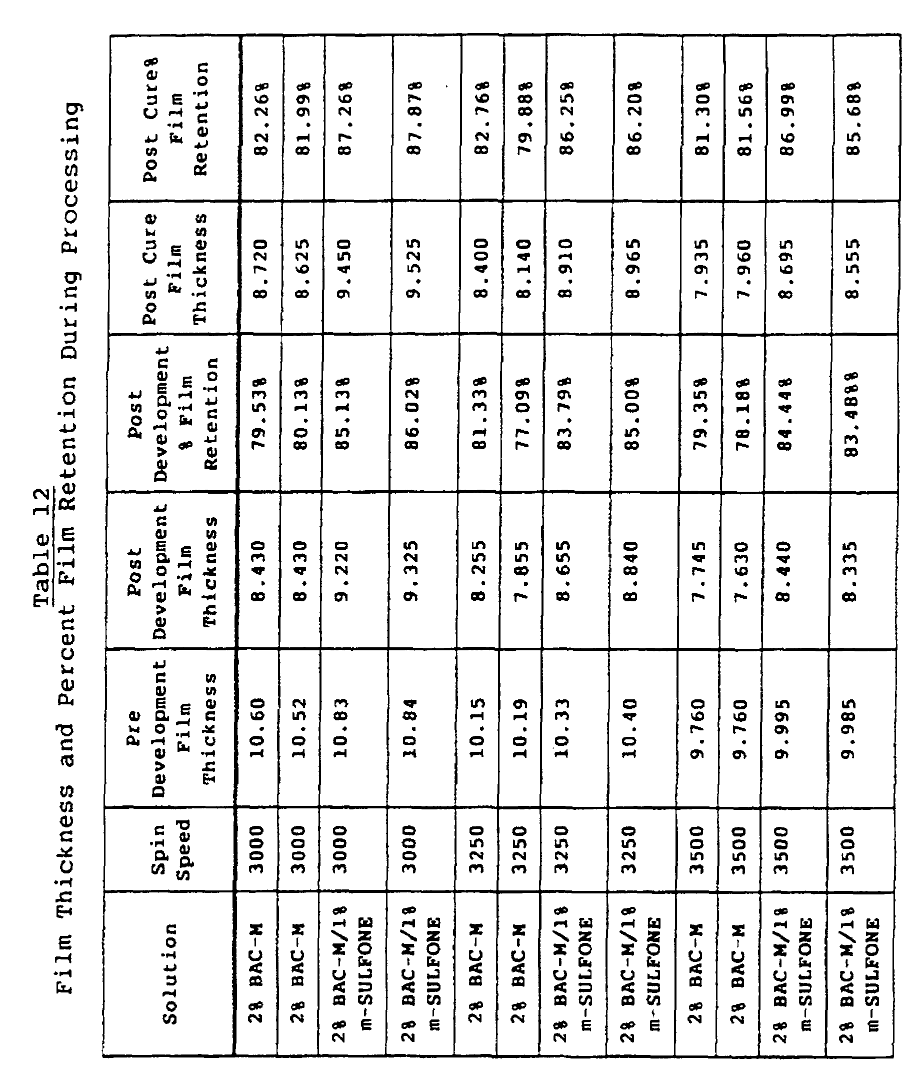

The most preferred resin for obtaining films with good film retention after development and cure is the resin containing 1 percent m-azidophenyl sulfone and 2 percent BAC-M which has a film retention of 85.13percent after development and 87.26.percent after cure for a starting film thickness of 10.83 microns.

### Example 20 Use of a Bisazide with Extended Conjugation

2,6-Bis[3-(4-azidophenyl)-2-propenylidene]-4]methylcyclohexanone of the structure: 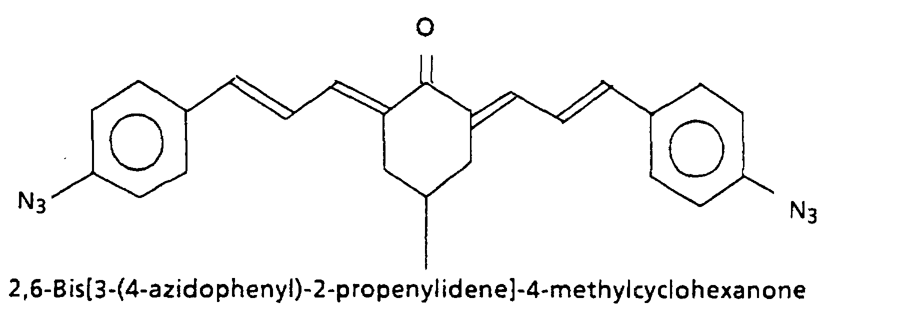 (0.110 g, 2 wt percent based on polymer) was added to an ambered vial followed by N-methyl pyrrolidone (2.118 g). The vial was capped and shaken by hand in order to dissolve the material. DVS-bis-BCB (10.019 g, 54.5 percent solids, 5.461 g polymer, adjusted percent solids: 45.0, molecular weight: Mn = 4500 g mol⁻¹, Mw = 59,000 g mol⁻¹) was added and the mixture was heated at 60°C for 18 min. in a water bath shaker while rotating at 150 rpm. The solution was mixed vigorously be shaking by hand for 1 min. After sonication to remove bubbles for 1 min, a homogeneous solution was obtained. The material was permitted to cool to room temperature.

Inside the cleanroom, the material was spin coated and processed using conditions similar to Example 20 with the following modifications:
1. A spin speed of 3500 rpm was used.
2. The material was exposed for 1000 mJ/cm² (measured at 365 nm, 17.9 s) followed by development in Stoddard solvent (immersion, 3 min). The film was dried using a stream of nitrogen followed by heating on a hot plate at 80°C for a few minutes to further dry the film.

Film thickness was determined after exposure and development or for the control substrate, after bake and exposure. The film thicknesses were also determined after the cure schedule used in Example 19.

| Film Thickness Results | |
|---|---|
| Control | |
| After bake and expose: | After development |
| 5.15 µm | 3.96 µm (77.0percent) |

| After cure | After cure |
|---|---|
| 5.59 µm | 3.925 µm (70.2percent) |

### Example 21: n-Butyl n-butyrate was a developing solvent

0.168 grams of 1,2-dihydro 2,2,4-trimethyl quinoline (PDTQ) was added to 33.281 grams of a 50 percent solids DVS-bis-BCB resin (molecular weight of Mn = 4,500 g/mole; Mw = 59,000 g/mole) solution in mesitylene and dissolved with heating at 60°C in a water bath shaker for 30 minutes. The solution was then filtered through a 5.0 micron filter into a clean amber bottle. 0.463 grams of BAC-M was added to the 30.099 grams of the filtered solution and dissolved with heating at 60°C in a water bath shaker for 30 minutes.

A clean silicon wafer was spin coated with a 0.5 percent aqueous solution of 3-aminopropyltriethoxysilane adhesion promoter at a spin speed of 3,500 rpms for 30 seconds. The BAC-M solution was then spin coated on the wafer using a 10 second spread cycle of 500 rpm followed by a spin speed of 3,500 rpm for 30 seconds to yield a 6.690 micron thick film. The wafer was placed in a nitrogen purged oven at 80°C for 30 minutes. After cooling to room temperature, the wafer was placed in an Oriel mask aligner and the photodefinable DVS-bis-BCB film on the wafer was then given an exposure dose of 1,000 mJ/cm² of light, measured at the 365 nm wavelength, (broad band source 350 to 450 nm) through a quartz photomask. The film thickness was measured with a profilometer to be 6.690 microns. The wafer was then immersed in butyl butyrate for 60 seconds and sprayed dry with a nitrogen stream. The wafer was placed in a nitrogen purged oven at 80°C for 10 minutes to further dry. After solvent development and drying, the film thickness was 5.105 microns as measured by a profilometer. The film was cured according to the following cure schedule:
50°C for 5 minutes
50°C to 100°C over 5 minutes
100°C for 15 minutes
100°C to 150°C over 15 minutes
150°C for 15 minutes
150°C to 250°C over 60 minutes
250°C for 60 minutes
250°C to 100°C over 120 minutes

The film thickness after cure was 5.170 microns as determined by a profilometer.

### Example 22: Fabrication of a multiple layer structure consisting of two alternating aluminum and photodefinable DVS-bis-BCB layers

1.006 grams of PDTQ was added to 199.358 grams of a 50 percent solids DVS-bis-BCB resin (molecular weight of Mn = 4,500 g/mole; Mw = 59,000 g/mole) solution in mesitylene and dissolved with heating at 60°C in a water bath shaker for 1 hour. 3.085 g of BAC-M was added to the solution and dissolved with heating at 60°C in a water bath shaker for 30 minutes. The solution was then filtered through a 0.5 micron filter into a clean 200 ml amber bottle.

A clean silicon wafer with a thermal silicon oxide layer was sputter coated with aluminum metal under typical metal deposition process conditions using a Leybold 560 Box Coater. The aluminum was deposited using a DC Magnetron sputtering in argon at 1500 watts for 60 minutes to yield a metal film of approximately 2.0 microns thick. The aluminum was patterned using Shipley Microposit 51400-37 positive photoresist. The Microposit 51400-37 photoresist was deposited on top of the aluminum by spin coating using a 3 second spread cycle at 500 rpms followed by a 30 second spin cycle at 2,500 rpm which produced a film thickness of approximately 4.0 microns. The photoresist was placed in a nitrogen purged oven at 100°C for 30 minutes. The wafer was then cooled to room temperature and then placed in a Canon PLA-501FA Mask Aligner which has a medium pressure Hg lamp as a light source. A photomask was placed on top of the wafer and the photoresist was exposed to the light at 405 nm wavelength for an exposure dose of 47 mJ/cm² The exposed positive photoresist was developed using Shipley Microposit 454 Developer (2 percent KOH). The development was done by immersing the substrates in a filtered, circulated developer bath at 18°C for 90 seconds. The patterned film was then dump-rinsed in Dl-water and spin-dried. The wafer was then flood exposed using the mask aligner light source for 236 mJ/cm² at the 405 nm wavelength. The wafer was placed in a nitrogen purged oven at 120°C for 30 minutes. The aluminum metal layer was etched by placing the wafer in an acid bath (48 percent DI-water, 43 percent phosphoric acid, 4.0 percent acetic acid, and 5.4 percent nitric acid) at 45°C for 15 minutes. The water was then dump-rinsed in Dl-water and spin-rinsed-dried to remove residual acid. The photoresist was stripped off of the substrate by placing the wafer on the spin coater and dynamically rinsing it with acetone, followed by methanol; the wafer was then spun until it was dry.

The clean wafer with patterned aluminum metal deposited on top of the thermal silicon oxide, was spin coated with a 0.5 percent aqueous solution of 3-aminopropyltriethoxysilane adhesion promoter at a spin speed of 2,500 rpms for 30 seconds. The photodefinable solution was then spin coated using a 10 second spread cycle of 500 rpms followed by a spin speed of 3,500 rpms for 30 seconds to yield a 6.7 micron thick film. The wafer was placed in a nitrogen purged oven at 80°C for 30 minutes. After cooling to room temperature, the wafer was placed in an Oriel mask aligner and the aluminum pattern on the wafer was aligned to the photomask. The photodefinable DVS-bis-BCB film on the wafer was then given an exposure dose of 700 mJ/cm² of light measured at the 365 nm wavelength (broad band source 350 to 450 nm). The exposed film was developed by immersion into 2-methoxy ethyl ether for 90 seconds and sprayed dry with a nitrogen stream. The wafer was "softcured" at 210°C in a nitrogen purged oven under the following conditions:
50°C for 5 minutes
50°C to 100°C over 5 minutes
100°C for 15 minutes
100°C to 150°C over 15 minutes
150°C for 15 minutes
150°C to 210°C over 30 minutes
210°C for 30 minutes
210°C to 100°C over 120 minutes
The wafer was then oxygen plasma cleaned for 15 minutes, dump-rinsed in Dl-water, and spin-rinsed dried.

A second 2.0 micron thick aluminum layer was deposited on top of patterned photodefinable DVS-bis-BCB film and patterned using the same processing conditions as the first metal deposition and patterning. The wafer was then oxygen plasma cleaned for 15 minutes, dump-rinsed in DI-water, and spin-rinsed dried.

The wafer was spin coated with a 0.5 percent aqueous solution of 3--aminopropyltriethoxysilane adhesion promoter at a spin speed of 2,500 rpms for 30 seconds. A second photodefinable DVS-bis-BCB layer was spin coated on the second patterned aluminum layer at a spin speed of 3,500 rpms to give a film coating thickness of 6.7 microns. The wafer was placed in a nitrogen purged oven at 80°C for 30 minutes. After cooling to room temperature, the wafer was placed in an Oriet mask aiigner and the aluminum pattern on the wafer was aligned to the photomask. The second photodefinable film was given an exposure dose of 700 mJ/cm² measured at the 365 nm wavelength (broad band source 350 to 450 nm). The wafer was then immersed in 2-methoxy ethyl ether for 90 seconds and sprayed dry with a nitrogen stream. The wafer was placed in a nitrogen purged oven at 80°C for 10 minutes to further dry. The film on the wafer was cured according to the following cure schedule:
50°C for 5 minutes
50°C to 100°C over 5 minutes
100°C for 15 minutes
100°C to 150°C over 15 minutes
150°C for 15 minutes
150°C to 250°C over 60 minutes
250°C for 60 minutes
250°C to 100°C over 120 minutes
The final multilayered structure was of high quality with good adhesion between the polymer and aluminum layers.

## Claims

1. A photo-curable, organic-soluble mixture comprising at least one oligomerized cyclobutarene containing 80 wt-% or more of oligomers of a degree of polymerization of three and more of cyclobutarenes of the formula 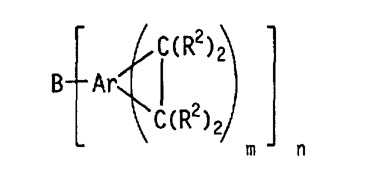 wherein B is a monovalent organic moiety, a direct bond, an n-valent bridging member comprising (1) a polyvalent inorganic moiety, or (2) a polyvalent organic moiety, or B is absent; Ar is a polyvalent aromatic or heteroaromatic moiety, an ar-poly-yl, having three or more valences, provided that two carbon atoms of the cyclobutane ring on the fused side are bonded to adjacent carbon atoms on the same aromatic ring of Ar, n is an integer of one or more and m is an integer of one or more and R² is a monovalent moiety, as its major component;
and at least one photosensitive agent in an amount sufficient to convert the mixture to an organic-insoluble solid upon exposing the mixture to photon radiation.

2. The photo-curable, organic-soluble mixture of claim 1, wherein the oligomerized cyclobutarene contains 90 wt-% or more of the oligomers.

3. The photo-curable, organic-soluble mixture of claim 1 or 2 wherein the oligomers have a molecular weight of 1,000 or more as determined from peak area percent by GPC, using a refractive index detector, uncorrected for response factors and as measured against polystyrene standards.

4. The photo-curable, organic-soluble mixture of any of the preceding claims, wherein the cyclobutarene is divinyltetramethyidisiloxane bisbenzocyclobutane.

5. The photo-curable, organic-soluble mixture of any of the preceding claims, wherein the photosensitive agent is selected from the group consisting of 2,6-bis(4-azidobenzylidene)-4-methylcyclohexanone, 2,6-bis[3-(4-azidophenyl)-2-propenylidene]-4]methylcyclohexanone and a mixture of 2,6-bis(4-azidobenzylidene)-4-methylcyclohexanone and m-azidophenyl sulfone.

6. The photo-curable, organic-soluble mixture of any of the preceding claims, wherein 1,2-dihydro-2,2,4-trimethylquinoline is present as antioxidant.

7. The photo-curable, organic-soluble mixture of any of the preceding claims, wherein a photosensitizer is present, selected from compounds represented by the following formulae: 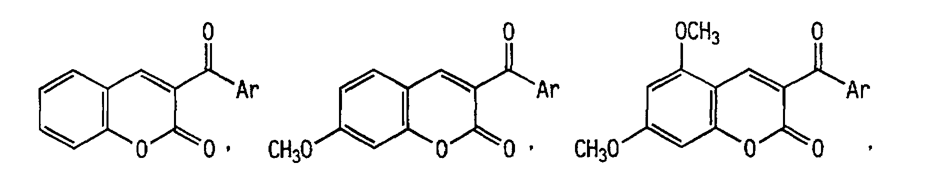 where Ar is represented by the following formulae: 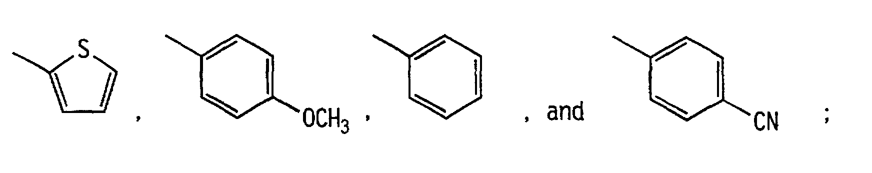 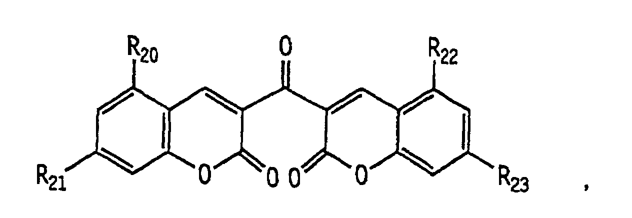 where R²⁰, R²¹, R²², R²³ are separately and independently H, OCH₃, and -N(C₂H₅)₂; 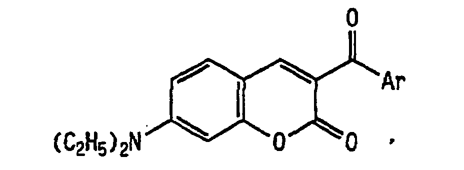 where Ar is represented by the following formulae: 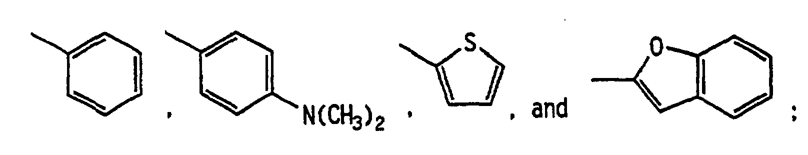 and 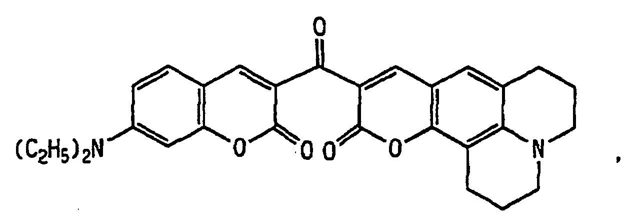 and

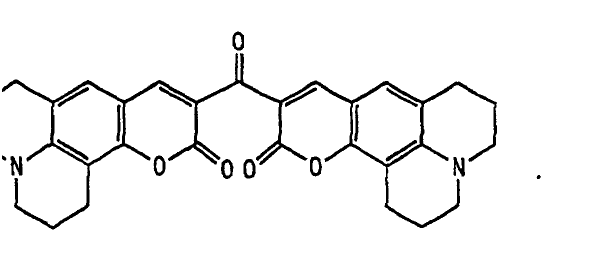

8. The photo-curable, organic-soluble mixture of claim 7, wherein the photosensitizer is
3,3'-carbonyl bis(7-diethylaminocoumarin) or
3,3'-carbonyl bis(7-methoxycoumarin).

9. A photo-cured pattern-coated substrate obtainable by applying the mixture of any of claims 1 to 8 to a substrate, exposing only a portion of the applied mixture to a curing amount of photon radiation and then treating the substrate with an organic developing solvent to remove the uncured portion of the mixture.

10. The coated substrate of claim 9, wherein the photo-cured pattern-coated substrate was exposed to a thermal cure.

11. The coated substrate of claims 9 or 10, which is an electronic device having at least one patterned dielectric layer which is the cured product of the mixture.

## Patentansprüche

1. Fotohärtbare, in organischen Lösungsmitteln lösliche Mischung, enthaltend wenigstens ein oligomerisiertes Cyclobutaren, das 80 Gew.-% oder mehr an Oligomeren eines Polymerisationsgrads von 3 oder mehr von Cyclobutarenen der Formel 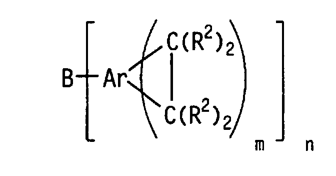 enthält, worin B eine einwertige organische Einheit, eine direkte Bindung, eine n-wertige verbrückende Gruppe, enthaltend (1) eine polyvalente anorganische Einheit oder (2) eine polyvalente organische Einheit, ist oder B nicht vorhanden ist; Ar eine polyvalente aromatische oder heteroaromatische Einheit, ein Ar-Poly-yl mit drei oder mehr Valenzen ist, unter der Voraussetzung, dass zwei Kohlenstoffatome des Cyclobutanrings an der anellierten Seite an benachbarten Kohlenstoffatomen desselben aromatischen Rings von Ar gebunden sind; n eine ganze Zahl von 1 oder mehr ist und m eine ganze Zahl von 1 oder mehr ist und R² eine einwertige Einheit ist, als Hauptkomponente;
und wenigstens ein fotosensitives Mittel in einer Menge, die ausreicht, um die Mischung durch Aussetzen an Photonenstrahlung in einen in organischen Lösungsmitteln unlöslichen Feststoff umzuwandeln.

2. Fotohärtbare, in organischen Lösungsmitteln lösliche Mischung nach Anspruch 1, worin das oligomerisierte Cyclobutaren 90 Gew.-% oder mehr der Oligomere enthält.

3. Fotohärtbare, in organischen Lösungsmitteln lösliche Mischung nach Anspruch 1 oder 2, worin die Oligomere ein Molekulargewicht von 1.000 oder mehr, bestimmt als Bandenflächenprozent/GPC unter Verwendung eines Brechungsindexdetektors, nichtkorrigiert für Ansprechfaktoren und gemessen gegen Polystyrolstandards, aufweisen.

4. Fotohärtbare, in organischen Lösungsmitteln lösliche Mischung nach einem der vorstehenden Ansprüche, worin das Cyclobutaren Divinyltetramethyldisiloxanbisbenzocyclobutan ist.

5. Fotohärtbare, in organischen Lösungsmitteln lösliche Mischung nach einem der vorstehenden Ansprüche, worin das fotosensitive Mittel ausgewählt ist aus der Gruppe bestehend aus 2,6-Bis(4-azidobenzyliden)-4-methylcyclohexanon, 2,6-B1s[3-(4-azidophenyl)-2-propenyliden]-4]methylcyclohexanon und einer Mischung von 2,6-Bis-(4-azidobenzyliden)-4-methylcyclohexanon und m-Azidophenylsulfon.

6. Fotohärtbare, in organischen Lösungsmitteln lösliche Mischung nach einem der vorstehenden Ansprüche, worin 1,2-Dihydro-2,2,4-trimethylchinolin als Antioxidationsmittel vorhanden ist.

7. Fotohärtbare, in organischen Lösungsmitteln lösliche Mischung nach einem der vorstehenden Ansprüche, worin ein Fotosensibilisator vorhanden ist, ausgewählt aus Verbindungen, die durch die folgenden Formeln dargestellt sind: 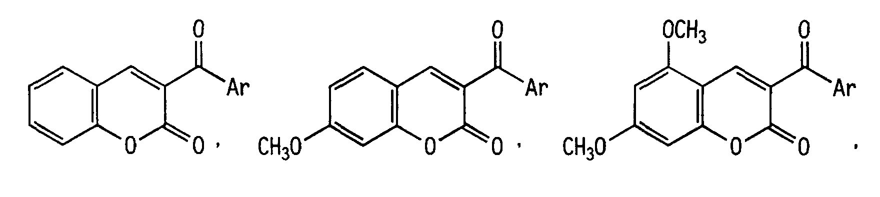 worin Ar durch die folgenden Formeln wiedergegeben ist: 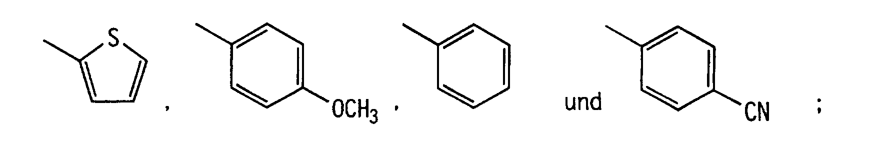 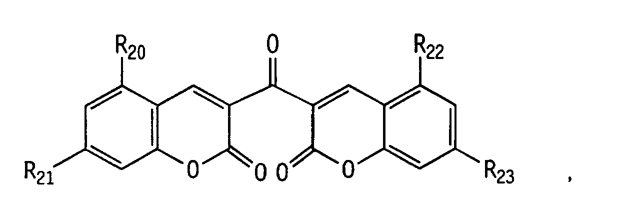 worin R²⁰, R²¹, R²², R²³ getrennt und unabhängig voneinander H, OCH₃, und -N(C₂H₅)₂ sind; 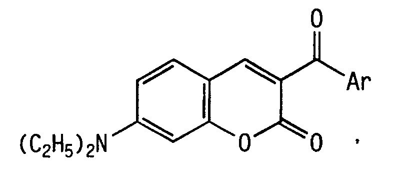 worin Ar durch die folgenden Formeln wiedergegeben ist: 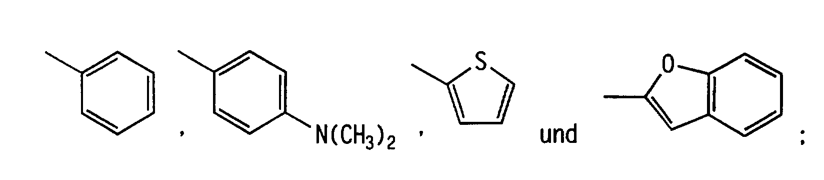 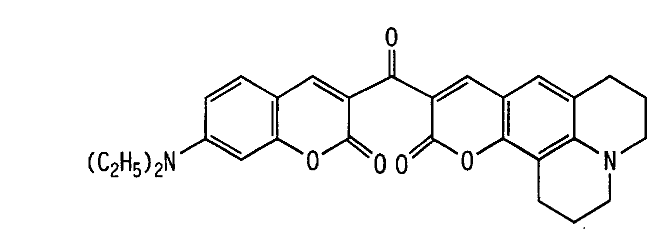 und

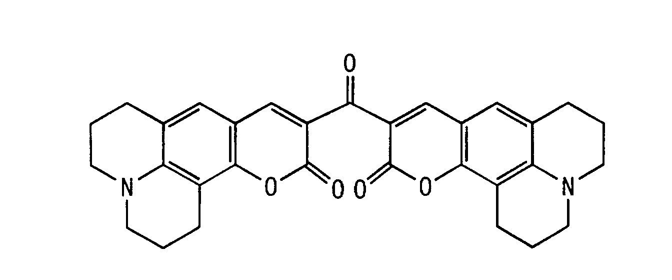

8. Fotohärtbare, in organischen Lösungsmitteln lösliche Mischung nach Anspruch 7, worin der Fotosensibilisator
3,3'-Carbonylbis(7-diethylaminocumarin) oder
3,3'-Carbonylbis(7-methoxycumarin) ist.

9. Fotogehärtetes, mit einem Muster beschichtetes Substrat, erhältlich durch Aufbringen der Mischung nach einem der Ansprüche 1 bis 8 auf ein Substrat, Belichten nur eines Teils der aufgebrachten Mischung mit einer härtenden Menge an Photonenstrahlung und dann Behandeln des Substrats mit einem organischen Entwicklungslösungsmittel, um den nichtgehärteten Teil der Mischung zu entfernen.

10. Beschichtetes Substrat nach Anspruch 9, wobei das fotogehärtete, mit einem Muster beschichtete Substrat einer thermischen Härtung ausgesetzt wurde.

11. Beschichtetes Substrat nach Ansprüchen 9 oder 10, das eine elektronische Vorrichtung mit wenigstens einer gedruckten dielektrischen Schicht ist, die das gehärtete Produkt der Mischung ist.

## Revendications

1. Mélange organosoluble photodurcissable, comprenant au moins un oligomère de cyclobutarène contenant au moins 80 % en poids d'oligomères, présentant un degré de polymérisation de 3 ou plus, de cyclobutarènes de formule : 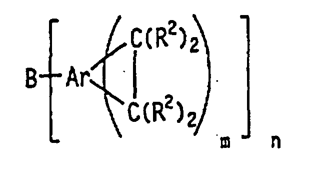 dans laquelle B représente un fragment organique monovalent, une liaison directe, un élément de liaison de valence n comprenant (1) un fragment inorganique polyvalent ou (2) un fragment organique polyvalent, ou B est absent ; Ar représente un fragment aromatique ou hétéroaromatique polyvalent, un ar-poly-yl, ayant trois valences ou plus, à condition que deux atomes de carbone du noyau cyclobutane du côté de la fusion soient liés à des atomes de carbone adjacents sur un même noyau aromatique de Ar ; n représente un nombre entier supérieur ou égal à 1 ; et m représente un nombre entier supérieur ou égal à 1 ; et R₂ représente un fragment monovalent, comme composant majoritaire ;
et au moins un agent photosensible en une quantité suffisante pour transformer le mélange en un solide insoluble dans les substances organiques lors d'une exposition du mélange à un rayonnement de photons.

2. Mélange organosoluble photodurcissable selon la revendication 1, dans lequel l'oligomère de cyclobutarène contient au moins 90 % en poids d'oligoméres.

3. Mélange organosoluble photodurcissable selon la revendication 1 ou 2, dans lequel les oligomères présentent une masse moléculaire de 1 000 ou plus, telle que déterminée à partir du pourcentage d'aire du pic par GPC, au moyen d'un détecteur d'indice de réfraction, non corrigé pour les facteurs de réponse, et tel que mesuré par rapport à des étalons de polystyrène.

4. Mélange organosoluble photodurcissable selon l'une quelconque des revendications précédentes, dans lequel le cyclobutarène est le divinyltétraméthyldisiloxane-bisbenzocyclobutane.

5. Mélange organosoluble photodurcissable selon l'une quelconque des revendications précédentes, dans lequel l'agent photosensible est choisi dans l'ensemble constitué par la 2,6-bis(4-azidobenzylidène)-4-méthylcyclohexanone, la 2,6-bis[3-(4-azidophényl)-2-propénylidène]-4-méthylcyclohexanone, et un mélange de 2,6-bis(4-azidobenzylidène)-4-méthylcyclohexanone et de m-azidophénylsulfone.

6. Mélange organosoluble photodurcissable selon l'une quelconque des revendications précédentes, dans lequel la 1,2-dihydro-2,2,4-triméthylquinoline est présente comme antioxydant.

7. Mélange organosoluble photodurcissable selon l'une quelconque des revendications précédentes, dans lequel un agent photosensible est présent, choisi parmi les composés représentés par les formules suivantes : 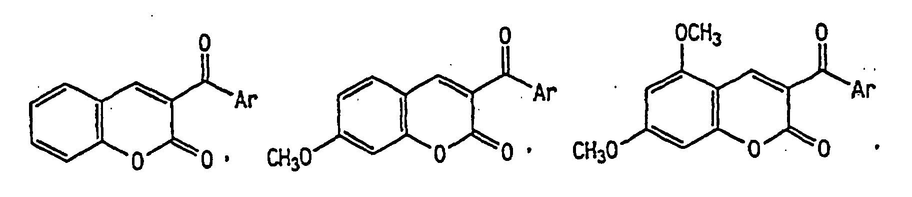 dans lesquelles Ar est représenté par les formules suivantes : 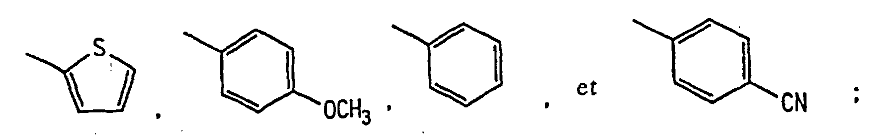 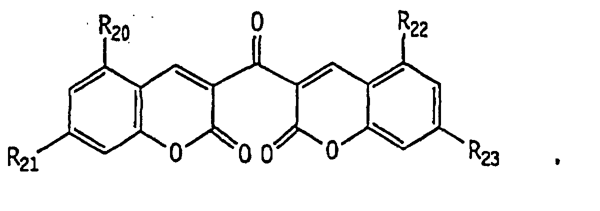 dans lesquelles R₂₀, R₂₁, R₂₂ et R₂₃ représentent séparément et indépendamment H, OCH₃ et -N(C₂H₅)₂; 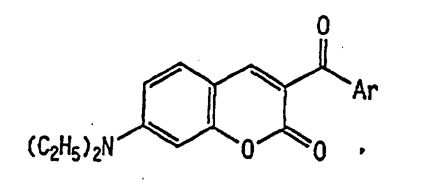 dans laquelle Ar est représenté par les formules suivantes : 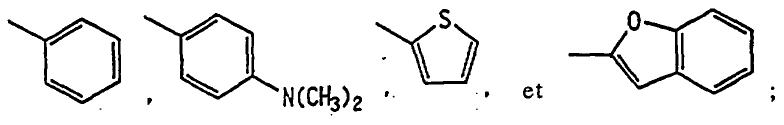 et 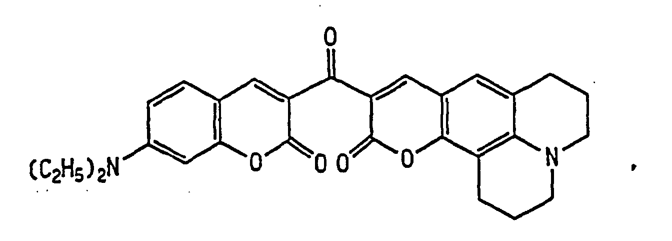 et

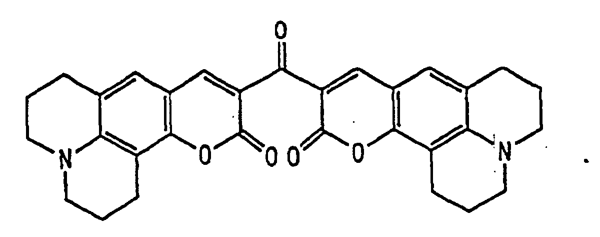

8. Mélange organosoluble photodurcissable selon la revendication 7, dans lequel l'agent photosensible est la 3,3'-carbonylbis(7-diéthylaminocoumarine) ou la 3,3'-carbonyl-bis(7-méthoxycoumarine).

9. Substrat revêtu d'un motif photodurci pouvant être obtenu par application d'un mélange selon l'une quelconque des revendications 1 à 8 sur un substrat, par exposition seulement d'une portion du mélange appliqué à une quantité de durcissement d'un rayonnement de photons, puis par traitement du substrat avec un solvant organique de développement pour éliminer la portion non durcie du mélange.

10. Substrat revêtu selon la revendication 9, dans lequel le substrat revêtu d'un motif photodurci a été exposé à un durcissement thermique.

11. Substrat revêtu selon la revendication 9 ou 10, qui est un dispositif électronique présentant au moins une couche diélectrique à motifs qui est le produit durci du mélange.
